# EUROPEAN PATENT SPECIFICATION

(11) **EP 1 984 388 B1**
(45) Date of publication and mention of the grant of the patent: **06.07.2016**
(21) Application number: 07750787.9
(22) Date of filing: 13.02.2007
(51) Int. Cl.: A61K 39/12, C12N 15/82, C07K 14/005, C12N 7/00, C12N 9/42

(54) **HPV ANTIGENS, VACCINE COMPOSITIONS, AND RELATED METHODS**
HPV-ANTIGENE, IMPFSTOFFZUSAMMENSETZUNGEN UND ZUGEHÖRIGE VERFAHREN
ANTIGENES DU PAPILLOMAVIRUS HUMAIN, COMPOSITIONS DE VACCIN ET METHODES

(30) Priority: 13.02.2006 US 773374 P
(43) Date of publication of application: 29.10.2008
(73) Proprietor: iBio, Inc., Newark, DE 19711 (US)
(72) Inventor: YUSIBOV, Vidadi, Havertown, PA 19083 (US); METT, Vadim, Newark, DE 19711 (US)
(74) Representative: Cornish, Kristina Victoria Joy
(86) International application number: PCT/US2007/003973
(87) International publication number: WO 2007/095320

(56) References cited:
- WO-A2-2004/043886
- WO-A2-2005/026375
- WO-A2-2005/026375
- WO-A2-2005/049839
- WO-A2-2005/081905
- WO-A2-2006/003018
- CZ-A3- 20 031 859
- US-A1- 2004 170 606
- US-B2- 6 797 491
- MICHAL SMAHEL ET AL: "Enhancement of immunogenicity of HPV16 E7 oncogene by fusion withE. coli [beta]-glucuronidase", THE JOURNAL OF GENE MEDICINE, vol. 6, no. 10, 1 October 2004 (2004-10-01), pages 1092-1101, XP55029806, ISSN: 1099-498X, DOI: 10.1002/jgm.596
- DANA POKORNÁ ET AL: "Combined immunization with fusion genes of mutated E7 gene of human papillomavirus type 16 did not enhance antitumor effect", THE JOURNAL OF GENE MEDICINE, vol. 7, no. 6, 1 June 2005 (2005-06-01), pages 696-707, XP55029973, ISSN: 1099-498X, DOI: 10.1002/jgm.733
- Vidadi Yusibov ET AL: "Novel approaches to the development of vaccines: progress on anthrax", Joint Meeting, 27-30 September 2005, Radisson SAS Royal Hotel, Bergen, Norway, 1 September 2005 (2005-09-01), page 13, XP55029750, Retrieved from the Internet: URL:http://www.sgm.ac.uk/meetings/pdfabstr acts/bergen2005abs.pdf [retrieved on 2012-06-13]
- YUSIBOV VIDADI ET AL: "The potential of plant virus vectors for vaccine production", DRUGS IN R & D, ADIS INTERNATIONAL, AUCKLAND, NZ, vol. 7, no. 4, 1 January 2006 (2006-01-01) , pages 203-217, XP009133148, ISSN: 1174-5886
- MARILLONNET SYLVESTRE ET AL: "Systemic Agrobacterium tumefaciens-mediated transfection of viral replicons for efficient transient expression in plants", NATURE BIOTECHNOLOGY, NATURE PUBLISHING GROUP, NEW YORK, NY, US LNKD- DOI:10.1038/NBT1094, vol. 23, no. 6, 1 June 2005 (2005-06-01), pages 718-723, XP002407902, ISSN: 1087-0156
- WAGNER B ET AL: "Plant virus expression systems for transient production of recombinant allergens in Nicotiana benthamiana", METHODS : A COMPANION TO METHODS IN ENZYMOLOGY, ACADEMIC PRESS INC., NEW YORK, NY, US LNKD- DOI:10.1016/J.YMETH.2003.08.005, vol. 32, no. 3, 1 March 2004 (2004-03-01), pages 227-234, XP004488972, ISSN: 1046-2023
- CANIZARES M C ET AL: "Use of viral vectors for vaccine production in plants", IMMUNOLOGY AND CELL BIOLOGY, CARLTON, AU LNKD- DOI:10.1111/J.1440-1711.2005.01339.X, vol. 83, no. 3, 1 June 2005 (2005-06-01), pages 263-270, XP002344926, ISSN: 0818-9641
- SANTI L ET AL: "Protection confered by recombinant Yersinia pestis antigens produced by a rapid and highly scalable plant expressoin system", PROCEEDINGS OF THE NATIONAL ACADEMY OF SCIENCES, NATIONAL ACADEMY OF SCIENCES, US, vol. 103, no. 4, 24 January 2006 (2006-01-24), pages 861-866, XP002407327, ISSN: 0027-8424, DOI: 10.1073/PNAS.0510014103
- DE JONG A ET AL: "Enhancement of human papillomavirus (HPV) type 16 E6 and E7-specific T-cell immunity in healthy volunteers through vaccination with TA-CIN, an HPV16 L2E7E6 fusion protein vaccine", VACCINE, ELSEVIER LTD, GB, vol. 20, no. 29-30, 4 October 2002 (2002-10-04), pages 3456-3464, XP004381809, ISSN: 0264-410X, DOI: 10.1016/S0264-410X(02)00350-X
- PETER J BALDWIN: "Vaccinia-Expressed Human Papillomavirus 16 and 18 E6 and E7 as a Therapeutic Vaccination for Vulval and Vaginal Intraepithelial Neoplasia", CLIN CANCER RES, vol. 9, 12 November 2003 (2003-11-12), pages 5205-5213, XP55030102,
- PIRUZIAN E.S. ET AL.: ' The use of a thermostable beta-glucanase gene from Clostridium thermocellum as a reporter gene in plants' MOL. GEN. GENET. vol. 257, no. 5, March 1998, pages 561 - 567, XP008130360
- SPILLIAERT R. ET AL.: 'Cloning and sequencing of a Rhodothermus marinus gene, bglA, coding for a thermostable beta-glucanase and its expression in Escherichia coli' EUR. J. BIOCHEM. vol. 224, no. 3, 15 September 1994, pages 923 - 930, XP000652194

## Description

### Related Applications

### Background of the Invention

Cervical cancer is the second most common cancer among women worldwide. Though screening has dramatically reduced the incidence of this disease in the developed world, in areas of the world where most women do not have access to regular gynecological care and screening, cervical cancer is second only to breast cancer as a cancer-related cause of death. Clinical, molecular and epidemiological investigations have identified human papilloma virus (HPV) as the major cause of cervical cancer and cervical dysplasia. Virtually all cervical cancers (about 99%) contain the genes of high-risk HPVs, most commonly types 16, 18, 31, and 45 (Ferlay *et al.,* 1999). About twelve percent (12%) of female cancers worldwide are due to HPV infections of the cervix. Every year, about 470,000 cases of cervical cancer are diagnosed worldwide, and nearly half of the women afflicted will die. It is estimated that HPV16 accounts for approximately 60% of cervical cancers, with HPV-18 adding another 10%-20%. Other high-risk types include types 31, 33, 35, 39, 45, 51, 52, 56, 58, 59, 68, and 73.

Moreover, HPV may play a role in certain carcinomas of the head and neck region as well as the more deadly melanomas and perhaps other cancers (see, *e.g.,* Mellin et al., 2000, Internat. J. Cancer, 89:300; Zumbach et al., 2000, Internat. J Cancer, 85:815; Dreau et al., 2000, Annals Surgery, 231:664; and Soini et al., 1996, Thorax, 51:887).

Current treatment of cervical dysplasia is limited to excisional or ablative procedures that remove or destroy cervical tissue. These procedures have efficacy rates of approximately 90% but are associated with morbidity and expense. Additionally, surgical treatments remove only the dysplastic tissue, leaving normal-appearing HPV-infected tissue untreated (Bell *et al.,* 2005)*.* It is therefore desirable to eradicate this infection using a vaccine. Preventive vaccination of adolescents before they first encounter HPV aims at this target. Some prophylactic vaccines are currently advancing in late phase clinical trials, with encouraging results. Due to the long latency period between infection and cancer, the benefits of a prophylactic vaccination, in terms of cancer incidence, would be visible after decades. However, already-infected individuals as well as patients suffering from advanced cancer could also benefit from therapeutic vaccinations. Vaccination against HPV to prevent infection and/or to treat malignant disease could substantially decrease morbidity and mortality from HPV-associated cancers. Thus, a remaining need exists to develop additional improved vaccines against HPV which are inexpensive and can be readily available to large populations. Furthermore, development of therapeutic vaccines against HPV able to hinder the progression of pre-existing lesions and malignant tumors, and even to eliminate them will be of extraordinary benefit to those afflicted with infection.

### Summary of the Invention

The present invention provides human papilloma virus (HPV) vaccines and vaccine components produced in plants. In some embodiments, one or more human papilloma virus antigens is generated as a fusion protein with a thermostable lickm protein. The present invention further comprises vaccine compositions containing HPV antigens. In some embodiments, inventive HPV vaccines comprise at least two different HPV antigens. Furthermore, the invention provides human papilloma virus (HPV) vaccines comprising at least two different human papilloma virus (HPV) antigens. Alternatively or additionally, inventive HPV vaccines may comprise one or more plant components. Still further provided are methods for production and use of the antigen and vaccine compositions of the invention.

### Brief Description of the Drawing

*Figure 1**.* Map of the pET32 plasmid. The top left indicates the region between the T7 promoter and the T7 terminator lacking in modified plasmid used for cloning target antigen.
*Figure 2**.* Production of the pET-PRACS-Lic-KDEL and pET-PRACS-Lic-VAC constructs from a modified pET 32 vector.
*Figure 3**.* Schematic of the pBI121 vector organization.
*Figure 4**.* Schematic organization of the derivation of pBID4 plasmid from a pBI vector after excision of the β-glucuronidase (GUS) gene and the addition of a TMV derived plasmid.
*Figure 5**.* Schematic of the fusion of E7 and E7GGG in lichenase sequence between BglII and HindIII sites and subsequent cloning in the pBID4 vector.
*Figures 6A-D*. Western analysis of *Agrobacterium* infiltrated plants expressing E7 constructs using an anti-lichenase antibody (6A,C) or anti-6HIS-E7 antibody (6B,D).
*Figures 7A-D*. Western analysis *of Agrobacterium* infiltrated plants expressing E7 constructs using an anti-lichenase antibody (7A,C) or anti-6HIS-E7 antibody (7B,D).
*Figure 8**.* Lichenase activity analysis of *Agrobacterium* infiltrated plants expressing E7 (8A, 8B) or E7GGG (8C, 8D) constructs using an anti-lichenase antibody.
*Figures 9A-D*. (9A) Western analysis of *Agrobacterium* infiltrated plants expressing E7GGG constructs using an anti-lichenase antibody. (9B-D) Coomassie staining analysis of protein fractions from isolation through purification procedures.

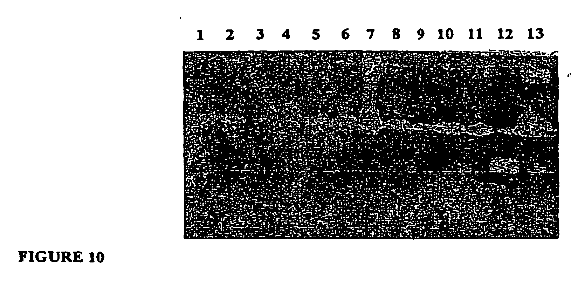

*Figure 10**.* Zymogram analysis performed on extracts from transgenic roots obtained from *Nicotiana benthamiana* leaf explants transformed with *Agrobacterium rhizogenes* containing pBID4-Lic-E7-KDEL (lanes1-9) and pBID4-Lic-E7GGG-KDEL constructs (lanes 10, 11); lane 12 = 150 ng lichenase (positive control); lane 13 = crude extract.from *Nicotiana benthamiana* leaves agro-infiltrated with *Agrobacterium rhizogenes* containing the pBID4-Lic-E7-KDEL construct.

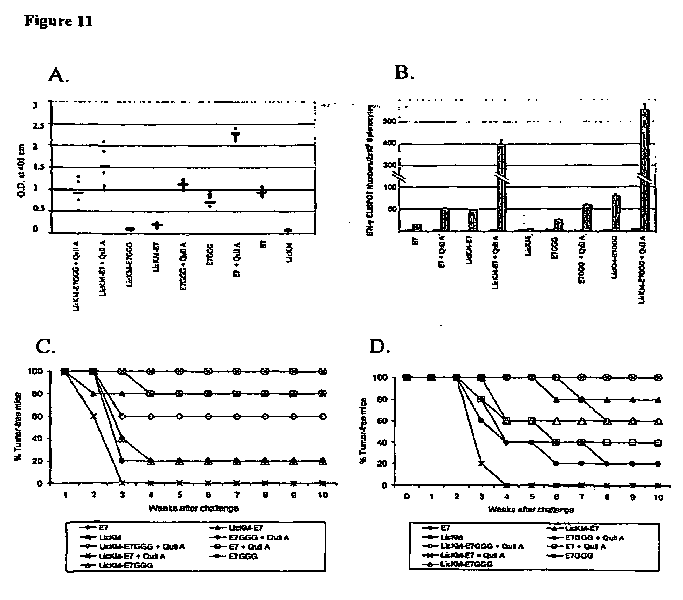

*Figures 11A-D*. Characterization and efficacy of plant-produced vaccine candidates. (11 A) E7-specific serum IgG responses. Data are presented as optical density values at 405 nm of 1:500 diluted sera. Data from individual animals are shown along with mean values. (11B) ELISPOT analysis of splenocytes from vaccinated mice. Data are presented as mean number of spots ± SD per 2x10⁵ splenocytes. Grey and black columns refer to cells stimulated with or without specific CTL E7 peptide, respectively. (11C) Prophylactic vaccination against TC-1-induced tumors. Data are represented as percentage of tumor-free mice. (11D) Therapeutic vaccination against TC-1-induced tumors. Data are represented as percentage of tumor-free mice.

### Detailed Description of the Invention

The invention relates to human papilloma virus (HPV) antigens useful in the preparation of vaccines against HPV infection, and fusion proteins comprising such HPV antigens operably linked to thermostable protein. The invention relates to methods of production of provided antigens in plant systems. Further, the invention relates to vectors, fusion proteins, plant cells, plants and vaccine compositions comprising the antigens and fusion proteins of the invention. Still further provided are methods of inducing immune response against HPV infection in a subject comprising administering vaccine compositions of the invention to a subject.

### HPV Antigens

Human papilloma virus (HPV) antigen proteins of the present invention include any immunogenic antigen protein or peptide capable of eliciting an immune response against HPV virus. Generally, immunogenic proteins of interest include HPV antigens *(e.g.,* E6 protein, E7 protein, *etc.),* an immunogenic portion thereof, and/or an immunogenic variant thereof.

HPV, antigens for use in accordance with the present invention may include full-length HPV proteins *(e.g.,* E6, E7, *etc.),* or fragments of such proteins, where such fragments retain immunological activity, and/or fusion proteins comprising such full-length HPV proteins or fragments.

HPV genes involved in transformation of cells *in vitro* are those encoding E6 and/or E7 (Bedell et al., 1987, J. Virol., 61:3635). Mechanisms by which the E6 and E7 proteins may cause cellular transformation have been proposed (Park et al., 1995, Cancer, 76:1902), and references cited therein). Based on their capacity to induce immunoprotective response against viral infection, E7 and E6 are the primary antigens of interest in generating vaccines. Additional HPV antigens may be useful in production of combination vaccines in order to improve efficacy of immunoprotection.

E6 is a small (approximately 15,000 MW) polypeptide comprising Zn-binding domains. A clue to its transforming function was provided by the observation that the protein binds p53. p53 protein is a well known tumor suppressor protein that negatively regulates cell cycle profession and consequently, cell growth and division. Binding of E6 to p53 results in ubiquitination and eventual degradation of the latter protein, which process involves another cellular protein termed "E6-associated protein." Consequently, cells expressing E6 will have a reduced basal level of p53. p53 levels are elevated in response to DNA damage. Such increased levels result in the enhanced expression of p21, an inhibitor of cyclin-dependent kinases, which protein mediates cell cycle arrest. This mechanism provides cells with a time window within which they can repair damaged DNA prior to its replication, which would result in the establishment of the damage/mutation. E6-mediated enhanced turnover of p53 may prevent the mechanism from operating. Recently, it was also found that E6 not only affects cell cycle regulation by virtue of accelerating degradation of p53, but also, more directly, by blocking p53 from interacting with DNA (Thomas et al., 1995, Oncogene, 10:261).

HPV E7 oncoprotein is a tumor-specific antigen and it is involved in malignant progression. E7 is a short-lived protein, which is degraded both *in vitro* and *in vivo* by the ubiquitin-proteasome pathway (Reinstein *et al.,* 2000)*.* E7 protein is a small (approximately 10,000 Mw), Zn-binding phosphoprotein capable of binding the retinoblastoma gene product Rb. Rb is a tumor suppressor binding to and inactivating transcription factor E2F. The latter factor controls transcription of a number of growth-related genes including those encoding thymidine kinase, c-myc, dihydrofolate reductase and DNA polymerase alpha. Rb-E2F complex formation prevents the expression of the latter genes in G0 and G1 phases of the cell cycle, restricting their expression to the S phase where the Rb-E2F complexes are programmed to dissociate, liberating active transcription factor E2F. Formation of Rb-E7 complexes prevents formation of Rb-E2F complexes with the result of shortening pre-S phases, *i.e.,* accelerating progression through the cell cycle. Attempts to produce large amounts of sequence-authentic, non-fused recombinant E7 protein in eukaryotic expression systems have practically failed, mainly due to its rapid degradation (Fernando *et al.,* 1999). Nevertheless, some E7-based HPV-specific therapeutic vaccines are currently being explored in phase II and III clinical trials. Preliminary results are promising, but still need further improvement by the association with more appropriate adjuvants able to stimulate efficacious cell mediated immunity (Frazer, 2004).

Correlative evidence for the importance of these mechanisms is provided by the observations that E6 proteins from highly oncogenic HPV types (*e.g*., HPV 16 and 18) have higher affinities for p53 than corresponding proteins from non-oncogenic types and that E7 proteins from highly oncogenic types have higher affinities for Rb than corresponding proteins from non-oncogenic types. Thus, E6 and E7 represent prime targets for the development of selective vaccine and anti-cancer therapy.

Thus, the invention provides plant cells and/or plants expressing a HPV antigen, which comprises E6, E7, a portion of E6, and/or a portion of E7 from HPV16 or HPV18 Full length nucleic acid and protein sequences for E7 and a modified E7 of one subtype are provided in SEQ ID NO.: 1, SEQ ID NO.: 2, SEQ ID NO.: 3, and SEQ ID NO.: 4.

While sequences of exemplary HPV antigens are provided herein, additional E6.and E7 sequences for various HPV strains and subtypes are known in the art and can be identified, for example in databases such as GenBank. Still further, activities and domains for each of E6 and E7 are known in the art. Thus, it will be appreciated that any sequence having the immunogenic characteristics of a domain of E6 and/or E7 may alternatively be employed. One skilled in the art will readily be capable of generating sequences having at least 75%, 80%, 85%, or 90% or more identity to the provided antigens. In certain embodiments, antigen sequences of HPV antigens comprise proteins include those having at least 95%, 96%, 97%, 98%, or more identity to sequences, or a portion thereof, wherein the antigen protein retains immunogenic activity. For example sequences having sufficient identity to HPV antigen(s) which retain immunogenic characteristics are capable of binding with antibodies which react with domains (antigen(s)) provided herein. Immunogenic characteristics often include three dimensional presentation of relevant amino acids or side groups. One skilled in the art can readily identify sequences with modest differences in sequence (*e.g.,* with difference in boundaries and/or some sequence alternatives, that, nonetheless preserve the immunogenic characteristics). For instance, sequences whose boundaries are near to (*e.g.,* within about 15 amino acids, 14 amino acids, 13 amino acids, 12 amino acids, 11 amino acids, 10 amino acids, 9 amino acids, 8 amino acids, 7 amino acids 6 amino acids, 5 amino acids 4 amino acids, 3 amino acids, 2 amino acids, or 1 amino acid) of the domain boundaries designated herein at either end of the designated amino acid sequence may be considered to comprise the relevant domain in accordance with the present invention. Thus, the invention contemplates use of a sequence of HPV antigen to comprise residues approximating the domain designation. For example, a domain of E7 has been engineered and expressed as an in-frame fusion protein as an antigen of the invention (see Examples herein). Further, one will appreciate that any domains, partial domains, or regions of amino acid sequence of HPV antigen (*e.g*., E6, E7) which are immunogenic can be generated using the constructs and methods provided herein. Still further, domains or subdomains can be combined, separately and/or consecutively for production of HPV antigens.

### Antigen Fusions with Thermostable Proteins

In certain aspects of the invention, provided are fusion polypeptide as defined in the claims which comprise an HPV antigen (or a fragment or variant thereof) operably linked to a thermostable protein. Inventive fusion polypeptides can be produced in in a plant or portion thereof (*e.g.,* plant, plant cell, root, sprout, *etc.*).

Enzymes or other proteins which are not found naturally in humans or animal cells are particularly appropriate for use in fusion polypeptides. Thermostable proteins that, when fused, confer thermostability to the fusion product are useful. Thermostability allows produced protein to maintain conformation, and maintain produced protein at room temperature This feature facilitates easy, time efficient and cost effective recovery of the fusion polypeptide. A representative family of thermostable enzymes is the glucanohydrolase family. These enzymes specifically cleave 1,4-β glucosidic bonds that are adjacent to 1,3-β linkages in mixed linked polysaccharides (Hahn et al., 1994, Proc. Natl. Acad. Sci., USA, 91:10417). The enzymes are found in cereals, such as oat and barley, and are also found in a number of fungal and bacterial species, including C. *thermocellum* (Goldenkova et al., 2002, Mol. Biol., 36:698). Thus, exemplary thermostable proteins for use in fusion polypeptides include glycosidase enzymes; exemplary thermostable glycosidase proteins include those represented by GenBank accession numbers selected from: P29716, P37073, P45798, P38645; P40942; P14002; 033830, 043097, P54583, P14288, 052629, P29094, P49067, JC7532, Q60037, P33558, P05117, P04954, Q4J929,O33833, P49425, P06279, P45703, P45702, P40943, P09961, Q60042, AAN05438, AAN05437, AAN05440, AAN05439, and AAD43138. Lichenase enzymes of use in fusion proteins include *Clostridium thermocellum* P29716, *Brevibacillus brevis* P37073, and *Rhodthermus marinus* P45798. Representative fusion proteins illustrated in the Examples utilize modified lichenase isolated from *Clostridium thermocellum.*

When designing fusion proteins and polypeptides in accordance with the invention, it is desirable, of course, to preserve the immunogenicity of the antigen. Still further, it is desirable in certain aspects of the invention to provide constructs which provide thermostability of the fusion protein. This feature facilitates easy, time efficient and cost effective recovery of the target antigen. In certain aspects, antigen fusion partners may be selected which provide additional advantages, including enhancement of immunogenicity, potential to incorporate multiple vaccine determinants, yet lack prior desirable. Two of these systems include production of clonal roots and clonal plant systems and derivatives thereof, as well as production of sprouted seedlings systems.

### Clonal Plant

Clonal roots maintain RNA viral expression vectors and stably produce target protein uniformly in the entire root over extended periods of time and multiple subcultures. In contrast to plants, where the target gene is eliminated via recombination during cell-to-cell or long distance movement, in root cultures the integrity of the viral vector is maintained and levels of target protein produced over time are similar to those observed during initial screening. Clonal roots allow for ease of production of material for oral formulation of antigen and vaccine compositions. Methods and reagents for generating a variety of clonal entities derived from plants which are useful for the production of antigen (*e.g.,* antigen proteins of the invention) have been described previously and are known in the art (see, for example, PCT Publication WO 05/81905. Clonal entities include clonal root lines, clonal root cell lines, clonal plant cell lines, and clonal plants capable of production of antigen (*e.g.,* antigen proteins of the invention). The invention further provides methods and reagents for expression of antigen polynucleotide and polypeptide products in clonal cell lines derived from various plant tissues (*e.g.,* roots, leaves), and in whole plants derived from single cells (clonal plants). Such methods are typically based on the use of plant viral vectors of various types.

For example, in one aspect, the invention provides methods of obtaining a clonal root line that expresses a polynucleotide encoding antigen of the invention comprising steps of: (i) introducing a viral vector that comprises a polynucleotide encoding antigen of the invention into a plant or portion thereof; and (ii) generating one or more clonal root lines from the plant. Clonal root lines may be generated, for example, by infecting a plant or plant portion (*e.g.,* a harvested piece of leaf) with an *Agrobacterium* (*e.g., A. rhizogenes*) that causes formation of hairy roots. Clonal root lines can be screened in various ways to identify lines that maintain virus, lines that express polynucleotides encoding antigen of the invention at high levels, *etc.* The invention further provides clonal root lines, *e.g.*, clonal root lines produced according to inventive methods, and further encompasses methods of expressing polynucleotides and producing polypeptides encoding antigen of the invention using the clonal root lines.

The invention provides methods of generating a clonal root cell line that expresses a polynucleotide encoding antigen of the invention comprising steps of: (i) generating a clonal root line, cells of which contain a viral vector whose genome comprises a polynucleotide encoding antigen of the invention; (ii) releasing individual. cells from the clonal root line; and (iii) maintaining the cells under conditions suitable for root cell proliferation. The invention provides clonal root cell lines and methods of expressing polynucleotides and producing polypeptides using clonal root cell lines.

In some embodiments, the invention provides methods of generating a clonal plant cell line that expresses a polynucleotide encoding antigen of the invention comprising steps of: (i) generating a clonal root line, cells of which contain a viral vector whose genome comprises a polynucleotide encoding antigen of the invention; (ii) releasing individual cells from the clonal root line; and (iii) maintaining the cells in culture under conditions appropriate for plant cell proliferation. The invention provides methods of generating a clonal plant cell line that expresses a polynucleotide encoding antigen of the invention comprising steps of: (i) introducing a viral vector that comprises a polynucleotide encoding antigen of the invention into cells of a plant cell line maintained in culture; and (ii) enriching for cells that contain the viral vector. Enrichment may be performed, for example, by (i) removing a portion of the cells from the culture; (ii) diluting the removed cells so as to reduce the cell concentration; (iii) allowing the diluted cells to proliferate; and (iv) screening for cells that contain the viral vector. Clonal plant cell lines may be used for production of an HPV antigen in accordance with the present invention.

The invention includes a number of methods for generating clonal plants, cells of which contain a viral vector that comprises a polynucleotide encoding antigen of the invention. For example, the invention provides methods of generating a clonal plant that expresses a polynucleotide encoding antigen of the invention comprising steps of: (i) generating a clonal root line, cells of which contain a viral vector whose genome comprises a polynucleotide encoding antigen of the invention; (ii) releasing individual cells from the clonal root line; and (iii) maintaining released cells under conditions appropriate for formation of a plant. The invention further provides methods of generating a clonal plant that expresses a polynucleotide encoding antigen of the invention comprising steps of: (i) generating a clonal plant cell line, cells of which contain a viral vector whose genome comprises a polynucleotide encoding antigen of the invention; and (ii) maintaining the cells under conditions appropriate for formation of a plant. In general, clonal plants according to the invention can express any polynucleotide encoding antigen of the invention. Such clonal plants can be used for production of an antigen-polypeptide.

As noted above, the present invention provides systems for expressing a polynucleotide or polynucleotides encoding antigen of the invention in clonal root lines, clonal root cell lines, clonal plant cell lines (*e.g.,* cell lines derived from leaf, stem, *etc.),* and/or in clonal plants. Polynucleotide-encoding antigen of the invention is introduced into an ancestral plant cell using a plant viral vector whose genome includes the polynucleotide encoding antigen of the invention operably linked to (*i.e.,* under control of) a promoter. A clonal root line or clonal plant cell line is established from a cell containing the virus according to any of several techniques further described below. The plant virus vector or portions thereof can be introduced into a plant cell by infection, by inoculation with a viral transcript or infectious cDNA clone, by electroporation, by T-DNA mediated gene transfer, *etc.*

The following sections describe methods for generating clonal root lines, clonal root cell lines, clonal plant cell lines, and clonal plants that express a polynucleotide encoding antigen of the invention are then described. A "root line" is distinguished from a "root cell line" in that a root line produces actual root-like structures or roots while a root cell line consists of root cells that do not form root-like structures. The use of the term "line" is intended to indicate that cells of the line can proliferate and pass genetic information on to progeny cells. Cells of a cell line typically proliferate in culture without being part of an organized structure such as those found in an intact plant. The use of the term "root line" is intended to indicate that cells in the root structure can proliferate without being part of a complete plant. It is noted that the term "plant cell" encompasses root cells. However, to distinguish the inventive methods for generating root lines and root cell lines from those used to directly generate plant cell lines from non-root tissue (as opposed to generating clonal plant cell lines from clonal root lines or clonal plants derived from clonal root lines), the terms "plant cell" and "plant cell line" as used herein generally refer to cells and cell lines that consist of non-root plant tissue. The plant cells can be, for example, leaf, stem, shoot, flower part, *etc.* It is noted that seeds can be derived from the clonal plants generated as derived herein. Such seeds will also contain a viral vector as will plants obtained from such seeds. Methods for obtaining seed stocks are well known in the art (see, *e.g.,* U.S. Patent Publication 2004/0093643).

### Clonal Root Lines

The present invention provides systems for generating a clonal root line in which-a-plant-viral-vector is-used-to direct expression of a polynucleotide.encoding antigen of the invention. One or more viral expression vector(s) including a polynucleotide encoding antigen of the invention operably linked to a promoter is introduced into a plant or a portion thereof according to any of a variety of known methods. For example, plant leaves can be inoculated with viral transcripts. Vectors themselves may be directly applied to plants (*e.g.,* via abrasive inoculations, mechanized spray inoculations, vacuum infiltration, particle bombardment, or electroporation). Alternatively or additionally, virions may be prepared (*e.g*., from already infected plants), and may be applied to other plants according to known techniques.

Where infection is to be accomplished by direct application of a viral genome to a plant, any available technique may be used to prepare the genome. For example, many viruses that are usefully employed in accordance with the present invention have ssRNA genomes. ssRNA may be prepared by transcription of a DNA copy of the genome, or by replication of an RNA copy, either *in vivo* or *in vitro.* Given the readily availability of easy-to-use *in vitro* transcription systems (*e.g.,* SP6, T7, reticulocyte lysate, *etc.*)*,* and also the convenience of maintaining a DNA copy of an RNA vector, it is expected that inventive ssRNA vectors will often be prepared by *in vitro* transcription, particularly with T7 or SP6 polymerase. Infectious cDNA clones can be used. Agrobacterially mediated gene transfer can be used to transfer viral nucleic acids such as viral vectors (either entire viral genomes or portions thereof) to plant cells using, *e.g.,* agroinfiltration, according to methods known in the art.

The plant or plant portion may then be then maintained (*e*.*g*., cultured or grown) under conditions suitable for replication of a viral transcript. In certain embodiments of the invention, virus spreads beyond an initially inoculated cell, *e.g.,* locally from cell to cell and/or systemically from an initially inoculated leaf into additional leaves. However, in some embodiments of the invention, virus does not spread. Thus, a viral vector may contain genes encoding functional MP and/or CP, but may be lacking one or both of such genes. In general, a viral vector is introduced into (infects) multiple cells in a plant or portion thereof.

Following introduction of a viral vector into the plant, leaves are harvested. In general, leaves may be harvested at any time following introduction of a viral vector. However, it may be desirable to maintain the plant for a period of time following introduction of a viral vector into the plant, *e.g.,* a period of time sufficient for viral replication and, optionally, spread of the virus from the cells into which it was initially introduced.-A clonal-root culture (or multiple cultures)-is prepared, *e.g*.,-by known methods further described below.

In general, any available method may be used to prepare a clonal root culture from a plant or plant tissue into which a viral vector has been introduced. One such method employs genes that exist in certain bacterial plasmids. These plasmids are found in various species of *Agrobacterium* that infect and transfer DNA to a wide variety of organisms. As a genus, *Agrobacteria* can transfer DNA to a large and diverse set of plant types including numerous dicot and monocot angiosperm species and gymnosperms (see Gelvin, 2003, Microbiol. Mol. Biol. Rev., 67:16, and references therein. The molecular basis of genetic transformation of plant cells is transfer from a bacterium and integration into a plant nuclear genome of a region of a large tumor-inducing (Ti) or rhizogenic (Ri) plasmid that resides within various *Agrobacterial* species. This region is referred to as the "T-region" when present in the plasmid and as "T-DNA" when excised from the plasmid. Generally, a single-stranded T-DNA molecule is transferred to a plant cell in naturally-occurring *Agrobacterial* infection and is ultimately incorporated (in double-stranded form) into the genome. Systems based on Ti plasmids are widely used for introduction of foreign genetic material into plants and for production of transgenic plants.

Infection of plants with various *Agrobacterial* species and transfer of the T-DNA has a number of effects. For example, *A. tumefaciens* causes crown gall disease while *A. rhizogenes* causes development of hairy roots at the site of infection, a condition known as "hairy root disease." Each root arises from a single genetically transformed cell. Thus, root cells in the roots are clonal, and each root represents a clonal population of cells. Roots produced by *A. rhizogenes* infection are characterized by a high growth rate and genetic stability (Giri et al., 2000, Biotech.Adv., 18:1, and references therein. In addition, such roots are able to regenerate genetically stable plants (Giri *et al.,* 2000, *supra*)*.*

In general, the present invention encompasses the use of any strain of *Agrobacteria* (*e.g.,* any *A. rhizogenes* strain) that is capable of inducing formation of roots from plant cells. As mentioned above, a portion of the Ri plasmid (Ri T-DNA) is responsible for causing hairy root disease. While transfer of this portion of the Ri plasmid to plant cells can conveniently be accomplished by infection with *Agrobacteria* harboring the Ri plasmid, the invention encompasses the use of several other methods of introducing the relevant region into a plant cell. Such methods include any available method of introducing genetic material into plant cells including, but not limited to, biolistics, electroporation, PEG-mediated DNA uptake, Ti-based vectors, *etc.* Relevant portions of the Ri T-DNA can be introduced into plant cells by use of a viral vector. Ri genes can be included in the same vector that contains a polynucleotide encoding antigen of the invention or in a different viral vector, which can be the same or a different type to that of vector that comprises the polynucleotide encoding antigen of the invention. It is noted that the entire Ri T-DNA may not be required for production of hairy roots, and the invention encompasses the use of portions of the Ri T-DNA, provided that such portions contain sufficient genetic material to induce root formation, as known in the art Additional genetic material, *e.g.,* genes present within the Ri plasmid but not within the T-DNA, may be transferred to a plant cell in accordance with the invention, particularly genes whose expression products facilitate integration of the T-DNA into plant cell DNA.

In order to prepare a clonal root line in accordance with certain embodiments of the invention, harvested leaf portions are contacted with *A. rhizogenes* under conditions suitable for infection and transformation. Leaf portions are maintained in culture to allow development of hairy roots. Each root is clonal, *i.e.,* cells in the root are derived from a single ancestral cell into which the Ri T-DNA was transferred. In accordance with the invention, a portion of such ancestral cells will contain a viral vector. Thus, cells in a root derived from such an ancestral cell will contain a viral vector since it will be replicated and will be transmitted during cell division. Thus, a high proportion, *(e.g.,* at least 50%, at least 75%, at least 80%, at least 90%, at least 95%), all (100%), or substantially all (at least 98%) of the cells will contain the viral vector. It is noted that since a viral vector is inherited by daughter cells within a clonal root, movement of a viral vector within the root is not necessary to maintain the viral vector throughout the root. Individual clonal hairy roots may be removed from a leaf portion and further cultured. Such roots are also referred to herein as root lines. Isolated clonal roots continue to grow following isolation.

A variety of different clonal root lines have been generated using the inventive methods. Root lines were generated using viral vectors containing polynucleotides encoding antigen of the invention (*e.g*., encoding, immunogenic peptide). Root lines were tested by Western blot. Root lines displayed a variety of different expression levels of various polypeptides. Root lines displaying high expression were selected and further cultured. Root lines were subsequently tested again and shown to maintain high levels of expression over extended periods of time, indicating stability. Levels of expression were comparable to or-greater than expression in intact plants infected with the same viral vector used to generate clonal root lines. In addition, stability of expression of the root lines was superior to that obtained in plants infected with the same viral vector. Up to 80% of such virus-infected plants reverted to wild type after 2 - 3 passages. (Such passages involved inoculating plants with transcripts, allowing the infection (local or systemic) to become established, taking a leaf sample, and inoculating fresh plants that are subsequently tested for expression.)

Root lines may be cultured on a large scale for production of antigen of the invention polypeptides as discussed further below. It is noted that clonal root lines (and cell lines derived from clonal root lines) can generally be maintained in medium that does not include various compounds, *e.g*., plant growth hormones such as auxins, cytokinins, *etc.,* that are typically employed in culture of root and plant cells. This feature reduces the expense associated with tissue culture, and the inventors expect that it will contribute significantly to the economic feasibility of protein production using plants.

Any of a variety of methods may be used to select clonal roots that express a polynucleotide encoding HPV antigen(s) of the invention. Western blots, ELISA assays, *etc.,* can be used to detect an encoded polypeptide. In the case of detectable markers such as GFP, alternative methods such as visual screens can be performed. If a viral vector comprising a polynucleotide that encodes a selectable marker is used, an appropriate selection can be imposed (*e.g.,* the leaf material and/or roots derived therefrom can be cultured in the presence of an appropriate antibiotic or nutritional condition and surviving roots identified and isolated). Certain viral vectors contain two or more polynucleotides encoding antigen of the invention, *e.g.,* two or more polynucleotides encoding different polypeptides. If one of these is a selectable or detectable marker, clonal roots that are selected or detected by selecting for or detecting expression of the marker will have a high probability of also expressing the second polynucleotide. Screening for root lines that contain particular polynucleotides can be performed using PCR and other nucleic acid detection methods.

Alternatively or additionally, clonal root lines can be screened for presence of the virus by inoculating host plants that will form local lesions as a result of virus infection (*e.g.,* hypersensitive host plants). For example, 5 mg of root tissue can be homogenized in 50 µl of phosphate buffer and used to inoculate a single leaf of a tobacco plant. If the virus is present in root cultures, within two to three days characteristic lesions will appear on the infected leaves. This means that the root line contains recombinant virus that carnes the polynucleotide encoding antigen of the invention (target gene). If no local lesions are formed, there is no virus, and the root line is rejected as negative. This method is highly time- and cost-efficient. After initially screening for the presence of virus, roots that contain the virus may be subjected to secondary screening, *e.g.,* by Western blot or ELISA to select high expressers. Additional screens, *e.g.,* screens for rapid growth, growth in particular media or under particular environmental conditions, *etc.,* can be applied. These screening methods may, in general, be applied in the development of any of the clonal root lines, clonal root cell lines, clonal plant cell lines, and/or clonal plants described herein.

As will be evident to one of ordinary skill in the art, a variety of modifications may be made to the description of the inventive methods for generating clonal root lines that contain a viral vector. Such modifications are within the scope of the invention. For example, while it is generally desirable to introduce the viral vector into an intact plant or portion thereof prior to introduction of the Ri T-DNA genes, in certain embodiments of the invention the Ri-DNA is introduced prior to introducing the viral vector. In addition, it is possible to contact intact plants with *A. rhizogenes* rather than harvesting leaf portions and then exposing them to the bacterium.

Other methods of generating clonal root lines from single cells of the plant or portion thereof that harbor a viral vector can be used (*i.e.,* methods not using *A. rhizogenes* or genetic material from the Ri plasmid). For example, treatment with certain plant hormones or combinations of plant hormones is known to result in generation of roots from plant tissue.

### Clonal Cell Lines Derived from Clonal Root Lines

As described above, the invention provides methods for generating clonal root lines, wherein cells in the root lines contain a viral vector. As is well known in the art, a variety of different cell lines can be generated from roots. For example, root cell lines can be generated from individual root cells obtained from the root using a variety of known methods. Such root cell lines may be obtained from various different root cell types within the root. In general, root material is harvested and dissociated (*e.g.*, physically and/or enzymatically digested) to release individual root cells, which are then further cultured. Complete protoplast formation is generally not necessary. If desired, root cells can be plated at very dilute cell concentrations, so as to obtain root cell lines from single root cells. Root cell lines derived in this manner are clonal root cell lines contain the viral vector. Such root cell lines therefore exhibit stable expression of the polynucleotide encodingantigen of the invention.-Clonal plant cell lines can be obtained in a similar manner from the clonal roots, *e.g.,* by culturing dissociated root cells in the presence of the appropriate plant hormones. Screens and successive rounds of enrichment can be used to identify cell lines that express the polynucleotide encoding antigen of the invention at high levels. However, if the clonal root line from which the cell line is derived already expresses at high levels, such additional screens may be unnecessary.

As in the case of the clonal root lines, cells of a clonal root cell line are derived from a single ancestral cell that contains the viral vector and will, therefore, also contain the viral vector since it will be replicated and will be transmitted during cell division. Thus a high proportion (*e.g*., at least 50%, at least 75%, at least 80%, at least 90%, at least 95%), all (100%), or substantially all (at least 98%) of the cells will contain the viral vector. It is noted that since the viral vector is inherited by daughter cells within the clonal root cell line, movement of the viral vector among the cells is not necessary to maintain the viral vector. The clonal root cell lines can be used for production of a polynucleotide encoding antigen of the invention as described below.

### Clonal Plant Cell Lines

The present invention provides methods for generating a clonal plant cell line in which a plant viral vector is used to direct expression of a polynucleotide encoding antigen of the invention. According to the inventive method, one or more viral expression vector(s) including a polynucleotide encoding an HPV antigen of the invention operably linked to a promoter is introduced into cells of a plant cell line that is maintained in cell culture. A number of plant cell lines from various plant types are known in the art, any of which can be used. Newly derived cell lines can be generated according to known methods for use in practicing the invention. A viral vector is introduced into cells of the plant cell line according to any of a number of methods. For example, protoplasts can be made and viral transcripts then electroporated into the cells. Other methods of introducing a plant viral vector into cells of a plant cell line can be used.

A method for generating clonal plant cell lines in accordance with the invention and a viral vector suitable for introduction into plant cells (*e.g.,* protoplasts) can be used as follows: Following introduction of the viral vector, the plant cell line may be maintained in tissue culture. During this time the viral vector may replicate, and polynucleotides encoding antigen of the invention may be expressed. Clonal plant cell lines are derived from the culture, *e.g*., by a process of successive enrichment. For example, samples may be removed from the culture, optionally with dilution so that the concentration of cells is low, and plated in Petri dishes in individual droplets. The droplets are then maintained to allow cell division.

It will be appreciated that the droplets may contain a variable number of cells, depending on the initial density of the culture and the amount of dilution. The cells can be diluted such that most droplets contain either 0 or 1 cell if it is desired to obtain clonal cell lines expressing the polynucleotide encoding antigen of the invention after only a single round of enrichment. However, it can be more efficient to select a concentration such that multiple cells are present in each droplet and then screen the droplets to identify those that contain expressing cells. In general, any appropriate screening procedure can be employed. For example, selection or detection of a detectable marker such as GFP can be used. Western blots or ELISA assays can be used. Individual droplets (100 µl) contain more than enough cells for performance of these assays. Multiple rounds of enrichment are performed to isolate successively higher expressing cell lines. Single clonal plant cell lines (*i.e.,* populations derived from a single ancestral cell) can be generated by further limiting dilution using standard methods for single cell cloning. However, it is not necessary to isolate individual clonal lines. A population containing multiple clonal cell lines can be used for expression of a polynucleotide encoding antigen of the invention.

In general, certain considerations described above for generation of clonal root lines apply to the generation of clonal plant cell lines. For example, a diversity of viral vectors containing one or more polynucleotides encoding antigen of the invention can be used as can combinations of multiple different vectors. Similar screening methods can be used. As in the case of the clonal root lines and clonal root cell lines, cells of a clonal plant cell line are derived from a single ancestral cell that contains the viral vector and will, therefore, also contain the viral vector since it will be replicated and will be transmitted during cell division. Thus a high proportion (*e.g.,* at least 50%, at least 75%, at least 80%, at least 90%, at least 95%), all (100%), or substantially all (at least 98%) of the cells will contain the viral vector. It is noted that since the viral vector is inherited by daughter cells within the clonal plant cell line, movement of the viral vector among the cells is not necessary to maintain the viral vector. The clonal plant cell line can be used for production of a polypeptide encoding antigen of the invention as described below.

### Clonal Plants

Clonal plants can be generated from the clonal roots, clonal root cell lines, and/or clonal plant cell lines produced according to the various methods described above. Methods for the generation of plants from roots, root cell lines, and plant cell lines such as the clonal root lines, clonal root cell lines, and clonal plant cell lines described herein are well known in the art (see, *e.g.,* Peres et al., 2001, Plant Cell, Tissue, and Organ Culture, 65:37 ). The invention therefore provides a method of generating a clonal plant comprising steps of (i) generating a clonal root line, clonal root cell line, or clonal plant cell line according to any of the inventive methods described above; and (ii) generating a whole plant from the clonal root line, clonal root cell line, or clonal plant. The clonal plants may be propagated and grown according to standard methods.

As in the case of the clonal root lines, clonal root cell lines, and clonal plant cell lines, the cells of a clonal plant are derived from a single ancestral cell that contains the viral vector and will, therefore, also contain the viral vector since it will be replicated and will be transmitted during cell division. Thus a high proportion (*e.g*., at least 50%, at least 75%, at least 80%, at least 90%, at least 95%), all (100%), or substantially all (at least 98%) of the cells will contain the viral vector. It is noted that since the viral vector is inherited by daughter cells within the clonal plant, movement of the viral vector is not necessary to maintain the viral vector.

### Sprouts and Sprouted Seedling Plant Expression Systems

Systems and reagents for generating a variety of sprouts and sprouted seedlings which are useful for the production of HPV antigen(s) according to the present invention have been described previously and are known in the art (see, for example, PCT Publication WO 04/43886). The present invention further provides sprouted seedlings, which may be edible, as a biomass containing an HPV antigen peptide or protein. In certain aspects, the biomass is provided directly for consumption of antigen compositions. In some aspects, the biomass is processed prior to consumption, for example, by homogenizing, crushing, drying, or extracting. In certain aspects, the HPV antigen is purified from the biomass and formulated into a pharmaceutical composition.

Additionally provided are methods for producirig HPV antigens in sprouted seedlings that can be consumed or harvested live (*e.g.,* sprouts, sprouted seedlings of the *Brassica* genus). In certain aspects, the present invention involves growing a seed to an -edible-sprouted seedling-in-a-contained, regulatable-environment (*e.g*., indoors, in a container, *etc.).* The seed can be a genetically engineered seed that contains an expression cassette encoding an HPV antigen, which expression is driven by an exogenously inducible promoter. A variety of exogenously inducible promoters can be used that are inducible, for example, by light, heat, phytohormones, nutrients, *etc.*

In related embodiments, the present invention provides methods of producing HPV antigen(s) in sprouted seedlings by first generating a seed stock for the sprouted seedling by transforming plants with an expression cassette that encodes HPV antigen using an *Agrobacrerium* transformation system, wherein expression of the HPV antigen is driven by an inducible promoter. Transgenic seeds can be obtained from the transformed plant, grown in a contained, regulatable environment, and induced to express the HPV antigen.

In some embodiments, methods are provided that involves infecting sprouted seedlings with a viral expression cassette encoding an HPV antigen, expression of which may be driven by any of a viral promoter or an inducible promoter. The sprouted seedlings are grown for two to fourteen days in a contained, regulatable environment, or at least until sufficient levels of the HPV antigen have been obtained for consumption or harvesting.

The present invention further provides systems for producing HPV antigen(s) in sprouted seedlings that include a housing unit with climate control and a sprouted seedling containing an expression cassette that encodes one or more HPV antigens, wherein expression is driven by a constitutive or inducible promoter. The inventive systems can provide unique advantages over the outdoor environment or greenhouse, which cannot be controlled. Thus the present invention enables the grower to precisely time the induction of expression of the HPV antigen. It can also greatly reduce the cost of producing HPV antigen(s).

In certain aspects, transiently transfected sprouts contain viral vector sequences encoding an inventive HPV antigen. Seedlings are grown for a time period so as to allow for production of viral nucleic acid in the sprout, followed by a period of growth wherein multiple copies of virus are produced, thereby resulting in production of antigen.

In certain aspects, genetically engineered seeds or embryos that contain a transgene encoding an HPV antigen are grown to the sprouted seedling stage in a contained, regulatable environment. The contained, regulatable environment may be a housing unit or room in which the seeds can be grown indoors. All environmental-factors of the contained, regulatable environment may be controlled. Since sprouts do not require light to grow, and lighting can be expensive, the genetically engineered seeds or embryos may be grown to the sprouted seedling stage indoors in the absence of light.

Other environmental factors that can be regulated in the contained, regulatable environment of the present invention include temperature, humidity, water, nutrients, gas *(e.g.,* 02 or CO2 content or air circulation), chemicals (small molecules such as sugars and sugar derivatives or hormones such as such as the phytohormones gibberellic or absisic acid, *etc.)* and the like.

According to certain methods of the present invention, expression of the transgene encoding an HPV antigen may be controlled by an exogenously inducible promoter. Exogenously inducible promoters are caused to increase or decrease expression of a transgene in response to an external, rather than an internal stimulus. A number of these environmental factors can act as inducers for expression of the transgenes carried by the expression cassettes of the genetically engineered sprouts. The promoter may be a heat-inducible promoter, such as a heat-shock promoter. For example, using as heat-shock promoter the temperature of the contained environment may simply be raised to induce expression of the transgene. Other promoters include light inducible promoters. Light-inducible promoters can be maintained as constitutive promoters if the light in the contained regulatable environment is always on. Alternatively or additionally, expression of the transgene can be turned on at a particular time during development by simply turning on the light. The promoter may be a chemically inducible promoter is used to induce expression of the transgene. According to these embodiments, the chemical could simply be misted or sprayed onto the seed, embryo, or seedling to induce expression of the transgene. Spraying and misting can be precisely controlled and directed onto the target seed, embryo, or seedling to which it is intended. The contained environment is devoid of wind or air currents, which could disperse the chemical away from the intended target, so that the chemical stays on the target for which it was intended.

According to the present invention, the time expression is induced can be selected to maximize expression of an HPV antigen in sprouted seedling by the time of harvest. Inducing expression in an embryo at a particular stage of growth, for example, inducing expression in an embryo at a particular number of days after germination, may result in maximum synthesis of the HPV antigen at the time of harvest. For example, inducing expression from the promoter 4 days after germination may result in more protein synthesis than inducing expression from the promoter after 3 days or after 5 days. Those skilled in the art will appreciate that maximizing expression can be achieved by routine experimentation. In some embodiments, the sprouted seedlings are harvested at about 1, 2, 3, 4, 5, 6, 7, 8, 9, 10, 11, or 12 days after germination.

In cases where the expression vector has a constitutive promoter instead of an inducible promoter, the sprouted seedling may be harvested at a certain time after transformation of the sprouted seedling. For example, if a sprouted seedling were virally transformed at an early stage of development, for example, at the embryo stage, the sprouted seedlings may be harvested at a time when expression is at its maximum post-transformation, *e.g.,* at about 1, 2, 3, 4, 5, 6, 7, 8, 9, 10, 11, 12, 13, or 14 days post-transformation. It could also be that sprouts develop one, two, three or more months post-transformation, depending on the germination of the seed.

Generally, once expression of HPV antigen begins, the seeds, embryos, or sprouted seedlings are allowed to grow until sufficient levels of HPV antigen are expressed. In certain aspects, sufficient levels are levels that would provide a therapeutic benefit to a patient if the harvested biomass were eaten raw. Alternatively or additionally, sufficient levels are levels from which the HPV antigen can be concentrated or purified from the biomass and formulated into a pharmaceutical composition that provides a therapeutic benefit to a patient upon administration. Typically, the antigen is not a protein expressed in the sprouted seedling in nature. At any rate, the HPV antigen is typically expressed at concentrations above that which would be present in the sprouted seedling in nature.

Once expression of the HPV antigen is induced, growth is allowed to continue until the sprouted seedling stage, at which time the sprouted seedlings are harvested. The sprouted seedlings can be harvested live. Harvesting live sprouted seedlings has several advantages including minimal effort and breakage. The sprouted seedlings of the present invention may be grown hydroponically, making harvesting a simple matter of lifting the sprouted seedling from its hydroponic solution. No soil is required for the growth of the sprouted seedlings of the invention, but may be provided if deemed necessary or desirable by the skilled artisan. Because sprouts can be grown without soil, no cleansing of sprouted seedling material is required at the time of harvest. Being able to harvest the sprouted seedling directly from its hydroponic environment without washing or scrubbing minimizes breakage of the harvested material. Breakage and wilting of plants induces apoptosis. During apoptosis, certain proteolytic enzymes become active, which can degrade the pharmaceutical protein expressed in the sprouted seedling, resulting in decreased therapeutic activity of the protein. Apoptosis-induced proteolysis can significantly decrease the yield of protein from mature plants. Using the methods of the present invention, apoptosis may be avoided when no harvesting takes place until the moment the proteins are extracted from the plant.

For example, live sprouts may be ground, crushed, or blended to produce a slurry of sprouted seedling biomass, in a buffer containing protease inhibitors. The buffer may be maintained at about 4°C. In some aspects, the sprouted seedling biomass is air-dried, spray dried, frozen, or freeze-dried. As in mature plants, some of these methods, such as air-drying, may result in a loss of activity of the pharmaceutical protein. However, because sprouted seedlings are very small and have a large surface area to volume ratio, this is much less likely to occur. Those skilled in the art will appreciate that many techniques for harvesting the biomass that minimize proteolysis of expressed protein are available and could be applied to the present invention.

In some embodiments, the sprouted seedlings are edible. In certain embodiments, sprouted seedlings expressing sufficient levels of HPV antigens are consumed upon harvesting (*e.g.,* immediately after harvest, within minimal period following harvest) so that absolutely no processing occurs before the sprouted seedlings are consumed. In this way, any harvest-induced proteolytic breakdown of the HPV antigen before administration of the HPV antigen to a patient in need of treatment is minimized. For example, sprouted seedlings that are ready to be consumed can be delivered directly to a patient. Alternatively or additionally, genetically engineered seeds or embryos are delivered to a patient in need of treatment and grown to the sprouted seedling stage by the patient. In one aspect, a supply of genetically engineered sprouted seedlings is provided to a patient, or to a doctor who will be treating patients, so that a continual stock of sprouted seedlings expressing certain desirable HPV antigens may be cultivated. This may be particularly valuable for populations in developing countries, where expensive pharmaceutical are not affordable or deliverable. The ease with which the sprouted seedlings of the invention can be grown makes the sprouted seedlings of the present invention particularly desirable for such developing populations.

The regulatable nature of the contained environment imparts advantages to the present invention over growing plants in the outdoor environment. In general, growing genetically engineered sprouted seedlings that express pharmaceutical proteins in plants provides a pharmaceutical product faster (because the plants are harvested younger) and with less effort, risk, and regulatory considerations than growing genetically engineered plants. The contained, regulatable environment used in the present invention reduces or eliminates the risk of cross-pollinating plants in the nature.

For example, a heat inducible promoter likely would not be used in the outdoors because the outdoor temperature cannot be controlled. The promoter would be turned on any time the outdoor temperature rose above a certain level. Similarly, the promoter would be turned off every time the outdoor temperature dropped. Such temperature shifts could occur in a single day, for example, turning expression on in the daytime and off at night. A heat inducible promoter, such as those described herein, would not even be practical for use in a greenhouse, which is susceptible to climatic shifts to almost the same degree as the outdoors. Growth of genetically engineered plants in a greenhouse is quite costly. In contrast, in the present system, every variable can be controlled so that the maximum amount of expression can be achieved with every harvest.

In certain embodiments, the sprouted seedlings of the present invention are grown in trays that can be watered, sprayed, or misted at any time during the development of the sprouted seedling. For example, the tray may be fitted with one or more watering, spraying, misting, and draining apparatus that can deliver and/or remove water, nutrients, chemicals *etc.* at specific time and at precise quantities during development of the sprouted seedling. For example, seeds require sufficient moisture to keep them damp. Excess moisture drains through holes in the trays into drains in the floor of the room. Typically, drainage water is treated as appropriate for removal of harmful chemicals before discharge back into the environment.

Another advantage of trays is that they can be contained within a very small space. Since no light is required for the sprouted seedlings to grow, the trays containing seeds, embryos, or sprouted seedlings may be tightly stacked vertically on top of one another, providing a large quantity of biomass per unit floor space in a housing facility constructed specifically for these purposes. In addition, the stacks of trays can be arranged in horizontal rows within the housing unit. Once the seedlings have grown to a stage appropriate for harvest (about two to fourteen days) the individual seedling trays are moved into a processing facility, either manually or by automatic means, such as a conveyor belt.

The system of the present invention is unique in that it provides a sprouted seedung biomass, which is a source of a HPV antigen. Whether consumed directly or processed into the form of a pharmaceutical composition, because the sprouted seedlings are grown in a contained, regulatable environment, the sprouted seedling biomass and/or pharmaceutical composition derived from the biomass can be provided to a consumer at low cost. In addition, the fact that the conditions for growth of the sprouted seedlings can be controlled makes the quality and purity of the product consistent. The contained, regulatable environment of the invention also obviates many safety regulations of the EPA that can prevent scientists from growing genetically engineered agricultural products out of doors.

### Transformed Sprouts

A variety of methods can be used to transform plant cells and produce genetically engineered sprouted seedlings. Two available methods for the transformation of plants that require that transgenic plant cell lines be generated *in vitro,* followed by regeneration of the cell lines into whole plants include *Agrobacterium tumefaciens* mediated gene transfer and microprojectile bombardment or electroporation. Viral transformation is a more rapid and less costly method of transforming embryos and sprouted seedlings that can be harvested without an experimental or generational lag prior to obtaining the desired product. For any of these techniques, the skilled artisan would appreciate how to adjust and optimize transformation protocols that have traditionally been used for plants, seeds, embryos, or spouted seedlings.

### Agrobacterium Transformation Expression Cassettes

*Agrobacterium* is a representative genus of the gram-negative family Rhizobiaceae. This species is responsible for plant tumors such as crown gall and hairy root disease. In dedifferentiated plant tissue, which is characteristic of tumors, amino acid derivatives known as opines are produced by the *Agrobacterium* and catabolized by the plant The bacterial genes responsible for expression of opines are a convenient source of control elements for chimeric expression cassettes. According to the present invention, *Agrobacterium* transformation system may be used to generate edible sprouted seedlings, which are merely harvested earlier than the mature plants. *Agrobacterium* transformation methods can easily be applied to regenerate sprouted seedlings expressing HPV antigens.

In general, transforming plants involves the transformation of plant cells grown in tissue culture by co-cultivation with an *Agrobacterium tumefaciens* carrying a plant/bacterial vector. The vector contains a gene encoding an HPV antigen. The *Agrobacterium* transfers the vector to the plant host cell and is then eliminated using antibiotic treatment. Transformed plant cells expressing the HPV antigen are selected, differentiated, and finally regenerated into complete plantlets (Hellens et al., 2000, Plant Mol. Biol, 42:819; Pilon-Smits et al., 1999, Plant Physiolog., 119:123; Barfield et al., 1991, Plant Cell Reports, 10:308; and Riva et al., 1998, J. Biotech, 1(3).

Expression vectors for use in the present invention include a gene (or expression cassette) encoding an HPV antigen designed for operation in plants, with companion sequences upstream and downstream of the expression cassette. The companion sequences are generally of plasmid or viral origin and provide necessary characteristics to the vector to transfer DNA from bacteria to the desired plant host.

The basic bacterial/plant vector construct may desirably provide a broad host range prokaryote replication origin, a prokaryote selectable marker. Suitable prokaryotic selectable markers include resistance toward antibiotics such as ampicillin or tetracycline. Other DNA sequences encoding additional functions that are well known in the art may be present in the vector.

*Agrobacterium* T-DNA sequences are required for *Agrobacterium* mediated transfer of DNA to the plant chromosome. The tumor-inducing genes of the T-DNA are typically removed and replaced with sequences encoding the HPV antigen. The T-DNA border sequences are retained because they initiate integration of the T-DNA region into the plant genome. If expression of the HPV antigen is not readily amenable to detection, the bacterial/plant vector construct may include a selectable marker gene suitable for determining if a plant cell has been transformed, *e.g.,* the nptII kanamycin resistance gene. On the same or different bacterial/plant vector (Ti plasmid) are Ti sequences. Ti sequences include the virulence genes, which encode a set of proteins responsible for the excision, transfer and integration of the T-DNA into the plant genome (Schell, 1987, Science, 237:1176). Other sequences suitable for permitting integration of the heterologous sequence into the plant genome may include transposon sequences, and the like, for homologous recombination.

Certain constructs will include an expression cassette encoding an antigen protein. One, two, or more expression cassettes may be used in a given transformation. The recombinant expression cassette contains, in addition to the HPV antigen encoding sequence, at least the following elements: a promoter region, plant 5' untranslated sequences, initiation codon (depending upon whether or not the expressed gene has its own), and transcription and translation termination sequences. In addition, transcription and translation terminators may be included in the expression cassettes or chimeric genes of the present invention. Signal secretion sequences that allow processing and translocation of the protein, as appropriate, may be included in the expression cassette. A variety of promoters, signal sequences, and transcription and translation terminators are described (see, for example Lawton et al., 1987, Plant Mol. Biol., 9:315; and U.S. Patent 5,888,789). In addition, structural genes for antibiotic resistance are commonly utilized as a selection factor (Fraley et al. 1983, Proc. Natl. Acad Sci., USA, 80:4803. Unique restriction enzyme sites at the 5' and 3' ends of the cassette allow for easy insertion into a pre-existing vector. Other binary vector systems for *Agrobacterium-*mediated transformation, carrying at least one T-DNA border sequence are described in PCT/EP99/07414 .

### Regeneration

Seeds of transformed plants may be harvested, dried, cleaned, and tested for viability and for the presence and expression of a desired gene product. Once this has been determined, seed stock is typically stored under appropriate conditions of temperature, humidity, sanitation, and security to be used when necessary. Whole plants may then be regenerated from cultured protoplasts (see, *e.g.,* Evans et al., Handbook of Plant Cell Cultures, Vol. 1, MacMillan Publishing Co., New York, 1983; and Vasil I.R. (ed.), Cell Culture and Somatic Cell Genetics of Plants, Acad. Press, Orlando, Vol. I, 1984, and Vol. III, 1986). In certain aspects, plants are regenerated only to the sprouted seedling stage. In some aspects, whole plants are regenerated to produce seed stocks and sprouted seedlings are generated from the seeds of the seed stock.

All plants from which protoplasts can be isolated and cultured to give whole, regenerated plants can be transformed by the present invention so that whole plants are recovered that contain the transferred gene. It is known that practically all plants can be regenerated from cultured cells or tissues, including, but not limited to, all major species of plants that produce edible sprouts. Some suitable plants include alfalfa, mung bean, radish, wheat, mustard, spinach, carrot, beet, onion, garlic, celery, rhubarb, a leafy plant such as cabbage or lettuce, watercress or cress, herbs such as parsley, mint, or clovers, cauliflower, broccoli, soybean, lentils, edible flowers such as sunflower *etc.*

Means for regeneration vary from one species of plants to the next. However, those-skilled in-the art will appreciate that generally a suspension of transformed protoplants containing copies of a heterologous gene is first provided. Callus tissue is formed and shoots may be induced from callus and subsequently rooted. Alternatively or additionally, embryo formation can be induced from protoplast suspension. These embryos germinate as natural embryos to form plants. Steeping a seed in water or spraying a seed with water to increase the moisture content of the seed to between 35-45% initiates germination. For germination to proceed, seeds are typically maintained in air saturated with water under controlled temperature and airflow conditions. Culture media will generally contain various amino acids and hormones, such as auxin and cytokinins. It is advantageous to add glutamic acid and proline to medium, especially for such species as alfalfa. Shoots and roots normally develop simultaneously. Efficient regeneration will depend on the medium, the genotype, and the history of the culture. If these three variables are controlled, then regeneration is fully reproducible and repeatable.

The mature plants, grown from transformed plant cells, are selfed and non-segregating, homozygous transgenic plants are identified. An inbred plant produces seeds containing inventive antigen-encoding sequences. Such seeds can be germinated and grown to the sprouted seedling stage to produce HPV antigen(s) according to the present invention.

In related embodiments, seeds of the present invention may be formed into seed products and sold with instructions on how to grow seedlings to the appropriate sprouted seedling stage for administration or harvesting into a pharmaceutical composition. In some related embodiments, hybrids or novel varieties embodying desired traits may be developed from inbred plants of the invention.

### Direct Integration

Direct integration of DNA fragments into the genome of plant cells by microprojectile bombardment or electroporation may be used in the present invention (see, *e.g.,* Kikkert et al., 1999, Plant: J. Tissue Cult. Assoc., 35:43; and Bates, 1994, Mol. Biotech, 2:135*).* More particularly, vectors that express HPV antigen(s) of the present invention can be introduced into plant cells by a variety of techniques. As described above, vectors may include selectable markers for use in plant cells. Vectors may include sequences that allow their selection and propagation in a secondary host, such as sequences containing an origin of replication and selectable marker. Typically, secondary hosts include bacteria and yeast. In one embodiment, the secondary host is bacteria (*e.g., Escherichia coli,* the origin of replication is a colEl-type origin of replication) and the selectable marker is a gene encoding ampicillin resistance. Such sequences are well known in the art and are commercially available *(e.g.,* Clontech, Palo Alto, CA or Stratagene, La Jolla, CA).

Vectors of the present invention may be modified to intermediate plant transformation plasmids that contain a region of homology to an *Agrobacterium tumefaciens* vector, a T-DNA border region from *Agrobacterium tumefaciens,* and antigen encoding nucleic acids or expression cassettes described above. Further vectors may include a disarmed plant tumor inducing plasmid *of Agrobacterium tumefaciens.*

According to this embodiment, direct transformation of vectors invention may involve microinjecting vectors directly into plant cells by the use of micropipettes to mechanically transfer recombinant DNA (see, *e.g.,* Crossway, 1985, Mol. Gen. Genet., 202:179). Genetic material may be transferred into a plant cell using polyethylene glycols (see, *e.g.,* Krens et al., 1982, Nature, 296:72). Another method of introducing nucleic acids into plants via high velocity ballistic penetration by small particles with a nucleic acid either within the matrix of small beads or particles, or on the surface (see, *e.g.,* Klein et al., 1987, Nature, 327:70; Knudsen et al., 1991, Planta, 185:330). Yet another method of introduction is fusion of protoplasts with other entities, either minicells, cells, lysosomes, or other fusible lipid-surfaced bodies (see, *e.g.,* Fraley et al., 1982, Proc. Natl. Acad Sci., USA, 79:1859). Vectors of the invention may be introduced into plant cells by electroporation (see, *e.g.,* Fromm et al. 1985, Proc. Natl. Acad. Sci., USA, 82:5824). According to this technique, plant protoplasts are electroporated in the presence of plasmids containing a gene construct. Electrical impulses of high field strength reversibly permeabilize biomembranes allowing introduction of plasmids. Electroporated plant protoplasts reform the cell wall divide and form plant callus, which can be regenerated to form sprouted seedlings of the invention. Those skilled in the art will appreciate how to utilize these methods to transform plants cells that can be used to generate edible sprouted seedlings.

### Viral Transformation

Similar to conventional expression systems, plant viral vectors can be used to produce full-length proteins, including full length antigen. According to the present invention, plant virus vectors may be used to infect and produce antigen(s) in seeds, embryos, sprouted seedlings, *etc.,* viral systems that can be used to express everything from short peptides to large complex proteins. Specifically, using tobamoviral vectors is described (see, for example, McCormick et al. 1999, Proc. Natl. Acad Sci., USA, 96:703; Kumagai-et al., 2000, Gene, 245:1.69; and Verch et al., 1998,. J. Immunol. Methods, 220:69). Thus, plant viral vectors have a demonstrated ability to express short peptides as well as large complex proteins.

In certain embodiments, transgenic sprouts, which express HPV antigen, are generated utilizing a host/virus system. Transgenic sprouts produced by viral infection provide a source of transgenic protein that has already been demonstrated to be safe. For example, sprouts are free of contamination with animal pathogens. Unlike, for example, tobacco, proteins from an edible sprout could at least in theory be used in oral applications without purification, thus significantly reducing costs. In addition, a virus/sprout system offers a much simpler, less expensive route for scale-up and manufacturing, since transgenes are introduced into virus, which can be grown up to a commercial scale within a few days. In contrast, transgenic plants can require up to 5-7 years before sufficient seeds or plant material is available for large-scale trials or commercialization.

According to the present invention, plant RNA viruses have certain advantages, which make them attractive as vectors for foreign protein expression. The molecular biology and pathology of a number of plant RNA viruses are well characterized and there is considerable knowledge of virus biology, genetics, and regulatory sequences. Most plant RNA viruses have small genomes and infectious cDNA clones are available to facilitate genetic manipulation. Once infectious virus material enters a susceptible host cell, it replicates to high levels and spreads rapidly throughout an entire sprouted seedling (one to ten days post inoculation). Virus particles are easily and economically recovered from infected sprouted seedling tissue. Viruses have a wide host range, enabling use of a single construct for infection of several susceptible species. These characteristics are readily transferable to sprouts.

Foreign sequences can be expressed from plant RNA viruses, typically by replacing one of the viral genes with desired sequence, by inserting foreign sequences into the virus genome at an appropriate position, or by fusing foreign peptides to structural proteins of a virus. Moreover, any of these approaches can be combined to express foreign sequences by trans-complementation of vital functions of a virus. A number of different strategies exist as tools to express foreign sequences in virus-infected plants using tobacco mosaic virus (TMV), alfalfa mosaic virus (AlMV), and chimeras thereof.

The genome of AlMV is a representative of the Bromoviridae family of viruses and consists-of three genomic RNAs (RNAsm1-3) and subgenomic RNA (RNA4). Genomic RNAs1 and 2 encode virus replicase proteins P1 and 2, respectively. Genomic RNA3 encodes cell-to-cell movement protein P3 and coat protein (CP). CP is translated from subgenomic RNA4, which is synthesized from genomic RNA3, and is required to start infection. Studies have demonstrated the involvement of CP in multiple functions, including genome activation, replication, RNA stability, symptom formation, and RNA encapsidation (see *e.g.,* Bol et al., 1971, Virology, 46:73; Van Der Vossen et al., 1994, Virology 202:891; Yusibov et al., Virology, 208:405; Yusibov et al., 1998, Virology, 242:1; Bol et al., (Review, 100 refs.), 1999, J. Gen. Virol., 80:1089; De Graaff, 1995, Virology, 208:583; Jaspars et al., 1974, Adv. Virus Res., 19:37; Loesch-Fries, 1985, Virology, 146:177; Neeleman et al., 1991, Virology, 181:687; Neeleman et al., 1993, Virology, 196: 883; Van Der Kuyl et al., 1991, Virology, 183:731; and Van Der Kuyl et al., 1991, Virology, 185:496).

Encapsidation of viral particles is typically required for long distance movement of virus from inoculated to un-inoculated parts of a seed, embryo, or sprouted seedling and for systemic infection. According to the present invention, inoculation can occur at any stage of plant development. In embryos and sprouts, spread of inoculated virus should be very rapid. Virions of AlMV are encapsidated by a unique CP (24 kD), forming more than one type of particle. Size (30- to 60-nm in length and 18 nm in diameter) and shape (spherical, ellipsoidal, or bacilliform) of a particle depends on the size of the encapsidated RNA. Upon assembly, the N-terminus of the AlMV CP is thought to be located on the surface of the virus particles and does not appear to interfere with virus assembly (Bol et al., 1971, Virology, 6:73). Additionally, AlMV CP with an additional 38-amino acid peptide at its N-terminus forms particles *in vitro* and retains biological activity (Yusibov et al., 1995, J. Gen. Virol., 77:567).

AlMV has a wide host range, which includes a number of agriculturally valuable crop plants, including plant seeds, embryos, and sprouts. Together, these characteristics make AlMV CP an excellent candidate as a carrier molecule and AlMV an attractive candidate vector for expression of foreign sequences in a plant at the sprout stage of development. Moreover, upon expression from a heterologous vector such as TMV, AlMV CP encapsidates TMV genome without interfering with virus infectivity (Yusibov et al., 1997, Proc. Natl. Acad Sci., USA, 94: 5784 ). This allows for use of TMV as a carrier virus for AlMV CP fused to foreign sequences.

TMV, the prototype of the tobamoviruses, has a genome consisting of a single plus-sense RNA encapsidated with a 17.0 kD CP, which results in rod-shaped particles (300 nm in length). CP is the only structural protein of TMV and is required for encapsidation and long distance movement of virus in an infected host (Saito et al., 1990, Virology, 176:329). 183 and 126 kD proteins are translated from genomic RNA and are required for virus replication (Ishikawa et al., 1986, Nuc. Acids Res., 14:8291). 30 kD protein is the cell-to-cell movement protein of virus (Meshi et al., 1987, EMBO J., 6: 2557). Movement and coat proteins are translated from subgenomic mRNAs (Hunter et al., 1976, Nature, 260:759; Bruening et al., 1976, Virology, 71:498; and Beachy et al., 1976, Virology, 73:498.

Other methods of transforming plant tissues include transforming the flower of a plant. Transformation of Arabidopsis thaliana can be achieved by dipping plant flowers into a solution of *Agrobacterium tumefaciens* (Curtis et al., 2001, Transgenic Res., 10:363; and Qing et al., 2000, Molecular Breeding: New Strategies in Plant Improvement, 1:67). Transformed plants are formed in the population of seeds generated by the "dipped" plants. At a specific point during flower development, a pore exists in the ovary wall through which *Agrobacterium tumejaciens* gains access to the interior of the ovary. Once inside the ovary, *Agrobacterium tumefaciens* proliferates and transforms individual ovules (Desfeux et al., 2000, Plant Physiology, 123:895). Transformed ovules follow the typical pathway of seed formation within the ovary.

### Production and Isolation of Antigen

In general, standard methods known in the art may be used for culturing or growing plants, plant cells, and/or plant tissues of the invention (*e.g*., clonal plants, clonal plant cells, clonal roots, clonal root lines, sprouts, sprouted seedlings, plants, *etc.)* for production of antigen(s). A wide variety of culture media and bioreactors have been employed to culture hairy root cells, root cell lines, and plant cells (see, for example, Giri et al., 2000, Biotechnol. Adv., 18:1; Rao et al., 2002, Biotechnol. Adv., 20:101; and references in both of the foregoing). Clonal plants may be grown in any suitable manner.

In a certain embodiments, HPV antigens of the invention may be produced by any known method. In some embodiments, an HPV antigen is expressed in a plant or portion thereof. Proteins are isolated and purified in accordance with conventional conditions and techniques known in-the art. These-include methods such as extraction, precipitation, chromatography, affinity chromatography, electrophoresis, and the like. The present invention involves the purification and affordable scaling up of production of HPV antigen(s) using any of a variety of plant expression systems known in the art and provided herein, including viral plant expression systems described herein.

In many embodiments of the present invention, it will be desirable to isolate vaccine antigen products. Where a protein of the invention is produced from plant tissue(s) or a portion thereof, *e.g*., roots, root cells, plants, plant cells, that express them, methods described in further detail herein, or any applicable methods known in the art may be used for any of partial or complete isolation from plant material. Where it is desirable to isolate an expression product from some or all of plant cells or tissues that express it, any available purification techniques may be employed. Those of ordinary skill in the art are familiar with a wide range of fractionation and separation procedures (see, for example, Scopes et al., Protein Purification: Principles and Practice, 3rd Ed., Janson et al., 1993; Protein Purification: Principles, High Resolution Methods, and Applications, Wiley-VCH, 1998; Springer-Verlag, NY, 1993; and Roe, Protein Purification Techniques, Oxford University Press, 2001). Often, it will be desirable to render a product more than about 50%, 60%, 70%, 80%. 85%, 90%, 91%, 92%. 93%. 94%. 95%, 96%, 97%, 98%, or 99% pure. See, *e.g.,* U.S. Patents 6,740,740 and 6,841,659 for discussion of certain methods useful for purifying substances from plant tissues or fluids.

Those skilled in the art will also appreciate that a method of obtaining a desired vaccine products is by extraction. Plant material (*e.g.,* roots, leaves, *etc.)* may be extracted to remove desired products from residual biomass, thereby increasing concentration and purity of a product. Plants may be extracted in a buffered solution. For example, plant material may be transferred into an amount of ice-cold water at a ratio of one to one by weight that has been buffered with, *e.g*., phosphate buffer. Protease inhibitors can be added as required. Plant material can be disrupted by vigorous blending or grinding while suspended in buffer solution and extracted biomass removed by filtration or centrifugation. Product carried in solution can be further purified by additional steps or converted to a dry powder by freeze-drying or precipitation. Extraction can be carried out by pressing. Plants or roots can be extracted by pressing in a press or by being crushed as they are passed through closely spaced rollers. Fluids expressed from crushed plants or roots are collected and processed according to methods well-known in the art. Extraction by pressing allows for release of products in a more concentrated form. However, the overall yield of product may be lower than if a product were extracted in solution.

### Vaccines

The present invention provides pharmaceutical antigen proteins for therapeutic use, such as antigen protein(s) or an immunogenic portion(s) thereof active as a vaccine for therapeutic and/or prophylactic treatment of HPV infection. Further, the invention provides veterinary use, as such antigen protein or immunogenic portion thereof is active in veterinary applications. In certain embodiments, antigen(s) may be produced by plant(s) or portion thereof (*e.g.,* root, cell, sprout, cell line, plant, *etc.)* of the invention. In certain embodiments, provided HPV antigens are expressed in plants, plant cells, and/or plant tissues (*e.g*., sprouts, sprouted seedlings, roots, root culture, clonal cells, clonal cell lines, clonal plants, *etc*.), and can be used directly from a plant or partially purified or purified in preparation for pharmaceutical administration to a subject.

The present invention provides plants, plant cells, and plant tissues expressing antigen(s) that maintains pharmaceutical activity when administered to a subject in need thereof. In certain embodiments, subjects include vertebrates, (*e.g.*, mammals, such as humans). According to the present invention, subjects include veterinary subjects such as bovines, ovines, canines, felines, *etc.* In certain aspects, an edible plant or portion thereof (*e.g*., sprout, root) is administered orally to a subject in a therapeutically effective amount. In some aspects, one or more HPV antigen(s) is provided in a pharmaceutical preparation, as described herein.

Vaccine compositions of the invention comprise one or more HPV antigens. In certain embodiments, at least two HPV antigens of the invention are included in an administered vaccine composition.

According to the present invention, treatment of a subject with a vaccine antigen is intended to elicit a physiological effect. A vaccine protein may have healing curative or palliative properties against a disorder or disease and can be administered to ameliorate relieve, alleviate, delay onset of, reverse or lessen symptoms or severity of a disease or disorder. A vaccine antigen may have prophylactic properties and can be used to prevent or delay onset of a disease or to lessen severity of such disease, disorder, or pathological condition when it does emerge. A physiological effect elicited by treatment of a subject with antigen according to the present invention can include an effective immune response such that infection by an organism is thwarted.

In some embodiments, inventive vaccines are delivered by oral and/or mucosal routes. Oral and/or mucosal delivery has the potential to prevent the infection of mucosal tissues, the primary gateway of infection for many pathogens. Oral and/or mucosal delivery can prime systemic immune response. There has been considerable progress in the development of heterologous expression systems for the oral administration of antigens that stimulate the mucosal-immune system and can prime systemic immunity. Previous efforts at delivery of oral vaccine however, have demonstrated a requirement for considerable quantities of antigen in achieving efficacy. Thus, the economical production of large quantities of target antigens is a prerequisite for the creation of effective oral vaccines. The development of plants expressing antigens, including thermostable antigens, represents a more realistic approach to such diffculties.

The pharmaceutical preparations of the present invention can be administered in a wide variety of ways to the subject, such as, for example, orally, nasally, enterally, parenterally, intramuscularly or intravenously, rectally, vaginally, topically, ocularly, pulmonarily, or by contact application. In certain embodiments, an HPV antigen expressed in a plant or portion thereof is administered to a subject orally by direct administration of the plant to the subject. In some aspects, a vaccine protein expressed in a plant or portion thereof is extracted and/or purified, and used for the preparation of a pharmaceutical composition. It may be desirable to formulate such isolated products for their intended use (*e.g.,* as a pharmaceutical agent, vaccine composition, *etc.).* In some embodiments, it will be desirable to formulate the products together with some or all of the plant tissues that express them.

Where it is desirable to formulate the product together with the plant material, it will often be desirable to have utilized a plant that is not toxic to the relevant recipient *(e.g.,* a human or other animal). Relevant plant tissue (*e.g.,* cells, roots, leaves) may simply be harvested and processed according to techniques known in the art, with due consideration to maintaining activity of the expressed product. In certain embodiments of the invention, it is desirable to have expressed the vaccine antigen in an edible plant (and, specifically in edible portions of the plant) so that the material can subsequently be eaten. For instance, where the vaccine antigen is active after oral delivery (when properly formulated), it may be desirable to produce the antigen protein in an edible plant portion, and to formulate the expressed vaccine antigen for oral delivery together with the some or all of the plant material with which the protein was expressed.

Vaccine antigens provided may be formulated according to known techniques. For example, an effective amount of a vaccine product can be formulated together with one or more organic or inorganic, liquid or solid, pharmaceutically suitable carrier materials. A vaccine antigen produced according to the present invention may be employed in dosage forms such as tablets, capsules, troches, dispersions, suspensions, solutions, gelcaps, pills, caplets, creams, ointments, aerosols, powder packets, liquid solutions, solvents, diluents, surface active agents, isotonic agents, thickening or emulsifying agents, preservatives, and solid bindings, as long as the biological activity of the protein is not destroyed by such dosage form.

In general, the compositions may comprise any of a variety of different pharmaceutically acceptable carrier(s), adjuvant(s), or vehicle(s), or a combination of one or more such carrier(s), adjuvant(s), or vehicle(s). As used herein the language "pharmaceutically acceptable carrier, adjuvant, or vehicle" includes solvents, dispersion media, coatings, antibacterial and antifungal agents, isotonic and absorption delaying agents, and the like, compatible with pharmaceutical administration. Materials that can serve as pharmaceutically acceptable carriers include, but are not limited to sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening agents, flavoring agents, and perfuming agents, preservatives, and antioxidants can be present in the composition, according to the judgment of the formulator (see Remington's Pharmaceutical Sciences, Fifteenth Edition, E.W. Martin, Mack Publishing Co., Easton, PA, 1975). For example, the vaccine antigen product may be provided as a pharmaceutical composition by means of conventional mixing granulating dragee-making, dissolving, lyophilizing, or similar processes.

### Additional vaccine components

Inventive vaccines may include additionally any suitable adjuvant to enhance the immunogenicity of the vaccine when administered to a subject. For example, such adjuvant(s) may include, without limitation, extracts of *Quillaja saponaria* (QS), including purified subfractions of food grade QS such as Quil A and QS-21, alum, aluminum hydroxide, aluminum phosphate, MF59, Malp2, incomplete Freund's adjuvant; Complete Freund's adjuvant; 3 De-O-acylated monophosphoryl lipid A (3D-MPL). Further adjuvants may include immunomodulatory oligonucleotides, for example unmethylated CpG sequences as disclosed in WO 96/02555. Combinations of different adjuvants, such as those mentioned hereinabove, are contemplated as providing an adjuvant which is a preferential stimulator of TH1 cell response. For example, QS21 can be formulated together with 3D-MPL. The ratio of QS21:3D-MPL will typically be in the order of 1:10 to 10:1; 1:5 to 5:1; and often substantially 1:1. In some embodiments, the range for optimal synergy is 2.5:1 to 1:1 3D-MPL: QS21. Doses of purified QS extracts suitable for use in a human vaccine formulation are from 0.01 mg to 10 mg per kilogram of bodyweight.

It should be noted that certain thermostable proteins (*e.g.,* lichenase) may themselves demonstrate immunoresponse potentiating activity, such that use of such protein whether in a fusion with an HPV antigen or separately may be considered use of an adjuvant. Thus, inventive vaccine compositions may further comprise one or more adjuvants. Certain vaccine compositions may comprise two or more adjuvants. Furthermore, depending on formulation and routes of administration, certain adjuvants may be desired in particular formulations and/or combinations.

In certain situations, it may be desirable to prolong the effect of an inventive vaccine by slowing the absorption of one or more components of the vaccine product (e.g., protein) that is subcutaneously or intramuscularly injected. This may be accomplished by the use of a liquid suspension of crystalline or amorphous material with poor water solubility. The rate of absorption of the product then depends upon its rate of dissolution, which in turn, may depend upon size and form. Alternatively or additionally, delayed absorption of a parenterally administered product is accomplished by dissolving or suspending the product in an oil vehicle. Injectable depot forms are made by forming microcapsule matrices of the protein in biodegradable polymers such as polylactide-polyglycolide. Depending upon the ratio of product to polymer and the nature of the particular polymer employed, the rate of release can be controlled. Examples of biodegradable polymers include poly(orthoesters) and poly(anhydrides). Depot injectable formulations may be prepared by entrapping the product in liposomes or microemulsions, which are compatible with body tissues. Alternative polymeric delivery vehicles can be used for oral formulations. For example, biodegradable, biocompatible polymers such as ethylene vinyl acetate, polyanhydrides, polyglycolic acid, collagen, polyorthoesters, and polylactic acid, *etc.,* can be used. Antigen(s) or an immunogenic portions thereof may be formulated as microparticles, *e.g.,* in combination with a polymeric delivery vehicle.

Enterally administered preparations of vaccine antigens may be introduced in solid, semi-solid, suspension or emulsion form and may be compounded with any pharmaceutically acceptable carriers, such as water, suspending agents, and emulsifying agents. The antigens may be administered by means of pumps or sustained-release forms, especially when administered as a preventive measure, so as to prevent the development of disease in a subject or to ameliorate or delay an already established disease. Supplementary active compounds, *e.g.,* compounds independently active against the disease or clinical condition to be treated, or compounds that enhance activity of an inventive compound, can be incorporated into or administered with the compositions. Flavorants and coloring agents can be used.

Inventive vaccine products, optionally together with plant tissue, are particularly well suited for oral administration as pharmaceutical compositions. Oral liquid formulations can be used and may be of particular utility for pediatric populations. Harvested plant material may be processed in any of a variety of ways (*e.g.,* air drying, freeze drying, extraction *etc*.), depending on the properties of the desired therapeutic product and its desired form. Such compositions as described above may be ingested orally alone or ingested together with food or feed or a beverage. Compositions for oral administration include plants; extractions of the plants, and proteins purified from infected plants provided as dry powders, foodstuffs, aqueous or non-aqueous solvents, suspensions, or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medial parenteral vehicles including sodium chloride solution, Ringer's dextrose solution, dextrose plus sodium chloride solution, Ringer's solution containing lactose or fixed oils. Examples of dry powders include any plant biomass that has been dried, for example, freeze dried, air dried, or spray dried. For example, the plants may be air dried by placing them in a commercial air dryer at about 120 degrees Fahrenheit until the biomass contains less than 5% moisture by weight. The dried plants may be stored for further processing as bulk solids or further processed by grinding to a desired mesh sized-powder. Alternatively or additionally, freeze-drying may be used for products that are sensitive to air-drying. Products may be freeze dried by placing them into a vacuum drier and dried frozen under a vacuum until the biomass contains less than about 5% moisture by weight. The dried material can be further processed as described herein.

Plant-derived material may be administered as or together with one or more herbal preparations. Useful herbal preparations include liquid and solid herbal preparations. Some examples of herbal preparations include tinctures, extracts (e.g., aqueous extracts, alcohol extracts), decoctions, dried preparations (*e.g.,* air-dried, spray dried, frozen, or freeze-dried), powders (*e.g*., lyophilized powder), and liquid. Herbal preparations can be provided in any standard delivery vehicle, such as a capsule, tablet, suppository, liquid dosage, *etc.* Those skilled in the art will appreciate the various formulations and modalities of delivery of herbal preparations that may be applied to the present invention.

Inventive root lines, cell lines, plants, extractions, powders, dried preparations and purified protein or nucleic acid products, *etc.,* can be in encapsulated form with or without one or more excipients as noted above. Solid dosage forms such as tablets, dragees, capsules, pills, and granules can be prepared with coatings and shells such as enteric coatings, release controlling coatings and other coatings well known in the pharmaceutical formulating art. In such solid dosage forms the active agent may be mixed with at least one inert diluent such as sucrose, lactose or starch. Such dosage forms may comprise, as is normal practice, additional substances other than inert diluents, *e.g*., tableting lubricants and other tableting aids such a magnesium stearate and microcrystalline cellulose. In the case of capsules, tablets and pills, the dosage forms may comprise buffering agents. They may optionally contain opacifying agents and can be of a composition that they release the active ingredient(s) only, in a certain part of the intestinal tract, and/or in a delayed manner. Examples of embedding compositions that can be used include polymeric substances and waxes.

In some methods, a plant or portion thereof expressing a HPV antigen according to the present invention, or biomass thereof, is administered orally as medicinal food. Such edible compositions are typically consumed by eating raw, if in a solid form, or by drinking, if in liquid form. The plant material can be directly ingested without a prior processing step or after minimal culinary preparation. For example, the vaccine protein may be expressed in a sprout which can be eaten directly. For instance, vaccine antigens expressed in an alfalfa sprout, mung bean sprout, or spinach or lettuce leaf sprout, e*tc.* In one embodiments, plant biomass may-be processed and the material recovered after the processing step is ingested.

Processing methods useful in accordance with the present invention are methods commonly used in the food or feed industry. The final products of such methods typically include a substantial amount of an expressed antigen and can be conveniently eaten or drunk. The final product may be mixed with other food or feed forms, such as salts, carriers, favor enhancers, antibiotics, and the like, and consumed in solid, semi-solid, suspension, emulsion, or liquid form. Such methods can include a conservation step, such as, e.g., pasteurization, cooking, or addition of conservation and preservation agents. Any plant may be used and processed in the present invention to produce edible or drinkable plant matter. The amount of HPV antigen in a plant-derived preparation may be tested by methods standard in the art, *e.g.,* gel electrophoresis, ELISA, or Western blot analysis, using a probe or antibody specific for the product. This determination may be used to standardize the amount of vaccine antigen protein ingested. For example, the amount of vaccine antigen may be determined and regulated, for example, by mixing batches of product having different levels of product so that the quantity of material to be drunk or eaten to ingest a single dose can be standardized. The contained, regulatable environment of the present invention, however, should minimize the need to carry out such standardization procedures.

A vaccine protein produced in a plant cell or tissue and eaten by a subject may be preferably absorbed by the digestive system. One advantage of the ingestion of plant tissue that has been only minimally processed is to provide encapsulation or sequestration of the protein in cells of the plant. Thus, the product may receive at least some protection from digestion in the upper digestive tract before reaching the gut or intestine and a higher proportion of active product would be available for uptake.

Pharmaceutical compositions of the present invention can be administered therapeutically or prophylactically. The compositions may be used to treat or prevent a disease. For example, any individual who suffers from a disease or who is at risk of developing HPV infection may be treated. It will be appreciated that an individual can be considered at risk for developing a disease without having been diagnosed with any symptoms of the disease. For example, if the individual is known to have been, or to be intended to be, in situations with relatively high risk of exposure to HPV infection, that individual will be considered at risk for developing the disease. Similarly, if members of an individual's family, friends or partner have been diagnosed with HPV infection, the Individual may be considered to be at risk for developing the disease.

Liquid dosage forms for oral administration include, but are not limited to, pharmaceutically acceptable emulsions, microemulsions, solutions, suspensions, syrups, and elixirs. In addition to active agents, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring, and perfuming agents.

Compositions for rectal or vaginal administration may be suppositories or retention enemas, which can be prepared by mixing the compositions of this invention with suitable non-irritating excipients or carriers such as cocoa butter, polyethylene glycol or a suppository wax which are solid at ambient temperature but liquid at body temperature and therefore melt in the rectum or vaginal cavity and release the active protein.

Dosage forms for topical, transmucosal or transdermal administration of a vaccine composition of this invention include ointments, pastes, creams, lotions, gels, powders, solutions, sprays, inhalants or patches. The active agent, or preparation thereof, is admixed under sterile conditions with a pharmaceutically acceptable carrier and any needed preservatives or buffers as may be required. For transmucosal or transdermal administration, penetrants appropriate to the barrier to be permeated may be used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, antigen or an immunogenic portion thereof may be formulated into ointments, salves, gels, or creams as generally known in the art. Ophthalmic formulation, eardrops, and eye drops are contemplated as being within the scope of this invention. Additionally, the present invention contemplates the use of transdermal patches, which have the added advantage of providing controlled delivery of a vaccine protein to the body. Such dosage forms can be made by suspending or dispensing the vaccine product in the proper medium. Absorption enhancers can be used to increase the flux of the vaccine protein across the skin. The-rate can be controlled by either providing a rate controlling membrane or by dispersing the vaccine protein in a polymer matrix or gel.

Inventive compositions are administered in such amounts and for such time as is necessary to achieve the desired result In certain embodiments of the present invention a "therapeutically effective amount" of a pharmaceutical composition is that amount effective for treating, attenuating, or preventing a disease in a subject. Thus, the "amount effective to treat, attenuate, or prevent disease," as used herein, refers to a nontoxic but sufficient amount of the pharmaceutical composition to treat, attenuate, or prevent disease in any subject For example, the "therapeutically effective amount" can be an amount to treat, attenuate, or prevent infection *(e.g.,* viral infection, HPV infection), *etc.*

The exact amount required may vary from subject to subject, depending on the species, age, and general condition of the subject, the stage of the disease, the particular pharmaceutical mixture, its mode of administration, and the like. Anthrax antigens of the invention, including plants expressing antigen(s) and/or preparations thereof may be formulated in dosage unit form for ease of administration and uniformity of dosage. The expression "dosage unit form," as used herein, refers to a physically discrete unit of vaccine composition appropriate for the patient to be treated. It will be understood, however, that the total daily usage of the compositions of the present invention is typically decided by an attending physician within the scope of sound medical judgment. The specific therapeutically effective dose level for any particular patient or organism may depend upon a variety of factors including the severity or risk of infection; the activity of the specific compound employed; the specific composition employed; the age, body weight, general health, sex of the patient, diet of the patient, pharmacokinetic condition of the patient, the time of administration, route of administration, and rate of excretion of the specific compound employed; the duration of the treatment; drugs used in combination or coincidental with the vaccine composition employed; and like factors well known in the medical arts.

It will be appreciated that the pharmaceutical compositions of the present invention can be employed in combination therapies (*e.g*., combination vaccine therapies), that is, the pharmaceutical compositions can be administered concurrently with, prior to, or subsequent to, one or more other desired vaccination procedures. The particular combination of therapies (*e.g.*, vaccines, therapeutic treatment of HPV infection) to employ in a combination regimen will take into account compatibility of the desired therapeutics and/or procedures and the desired therapeutic effect to be achieved. It will be appreciated that the therapies and/or vaccines employed may achieve a desired effect for the same disorder (for example, an inventive compound may be administered concurrently with another HPV vaccine), or they may achieve different effects. In certain embodiments, vaccine compositions comprise at least two HPV antigens. For example, certain vaccine compositions can comprise at least two HPV antigens of the invention (*e.g.*, an E7 of HPV 16 domain and an E7 of HPV 18 domain containing antigen of the invention). In some aspects such combination vaccines may include one thermostable fusion protein comprising HPV antigen; in some aspects, two or more thermostable fusion proteins comprising HPV antigen are provided. In certain embodiments, multiple antigens will be derived from HPV of the same strain, and can include antigens derived from different proteins. In some embodiments, multiple antigens will be derived from HPV of the same strain and can include antigens derived from the same protein (*e.g*., different domains of the same protein). In certain embodiments, multiple antigens will be derived from HPV of different strains and include antigens derived of different proteins. Still further combinations contemplate use of multiple antigens derived from different strains and the same protein. Variations and/or combinations of the foregoing exemplary combinations are contemplated. For example in some embodiments, one E7, two E7 or three E7 domains from different strains are combined to comprise a vaccine composition of the invention. Where combination vaccines are utilized, it will be understood that any combination of HPV antigens may be used for such combinations.

### Kits

Disclosed is a pharmaceutical pack or kit including live sprouted seedlings, clonal entity or plant producing an HPV antigen according to the present invention, or preparations, extracts, or pharmaceutical compositions containing the vaccine in one or more containers filled with one or more of the ingredients of the pharmaceutical compositions of the invention. The pharmaceutical pack or kit includes an additional approved therapeutic agent (*e.g.*, HPV antigen, HPV vaccine) for use as a combination therapy. Optionally associated with such container(s) can be a notice in the form prescribed by a governmental agency regulating the manufacture, use or sale of pharmaceutical products, which notice reflects approval by the agency of manufacture, use, or sale for human administration.

Kits are disclosed that include therapeutic reagents. As but one non-limiting example, HPV vaccine can be provided as oral formulations and administered as therapy. Alternatively or additionally, HPV vaccine can be provided in an injectable formulation for administration. Pharmaceutical doses or instructions therefore may be provided in the kit for administration to an individual suffering from or at risk for HPV infection.

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. The following examples contain information, exemplification and guidance, which can be adapted to the practice of this invention in its various embodiments and the equivalents thereof.

### Exemplification

### Example 1. Generation of Vaccine Candidate Constructs

### Generation of antigen sequences from human papilloma virus

Because of safety concerns, the HPV16 E7 oncogene (KO2718, Seedorf et al., 1985, Virology, 145:181) already cloned into pQE30 (pQE30-E7) between BamHI / PstI restriction sites, was mutated using the Quikchange Site-Directed Mutagenesis Kit (Stratagene) to generate the plasmid pQE30-E7GGG. Three point mutations were introduced into the pRB-binding site of the E7 gene as indicated by the bolded oligonucleotides of the forward and backward "fast polynucleotide liquid chromatography"-purified primers designed to introduce the mutations, respectively:
*E7GGG dir:* 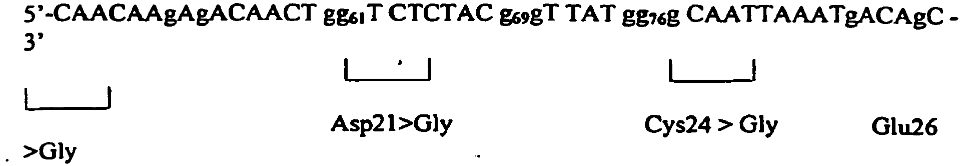
5'-CAACAAgAgACAACT gg61T CTCTAC g69gT TAT gg76g CAATTAAATgACAgC -
3'
Asp21>Gly | Cys24 > Gly | Glu26
>Gly
*E7CGG rev:*

5'-gCTgTCATTTAATTg CCC ATA ACC gTAgAg ACC AgTTgTCTCTggTTg C-3'
Glu26 >Gly Cys24 > Gly Asp21>Gly

The introduced mutations, resulted in the substitution of three amino acids in the E7 protein sequence: D21G (Asp21>Gly), C24G (Cys24 > Gly), E26G (Glu26 >Gly) abolishing the E7 protein transformation potential. The authenticity of the resultant gene, denoted E7GGG, was confirmed by sequencing
HPV16 E7 (KO2718, Seedorf *et al., supra*) (SEQ ID NO.: 1) 
ATGCATGGAGATACACCTACATTGCATGAATATATGTTAGATTTGCAACCAGA
GACAACTGATCTCTACTGTTATGAGCAATTAAATGACAGCTCAGAGGAGGAG
GATGAAATAGATGGTCCAGCTGGACAAGCAGAACCGGACAGAGCCCATTACA
ATATTGTAACCTTTTGTTGCAAGTGTGACTCTACGCTTCGGTTGTGCGTACAA
AGCACACACGTAGACATTCGTACTTTGGAAGACCTGTTAATGGGCACACTAG
GAATTGTGTGCCCCATCTGTTCTCAGAAACCATAA
E7 (SEQ ID NO.: 2): 
MHGDTPTLHEYMLDLQPETTDLYCYEQLNDSSEEEDEIDGPAGQAEPDRAHYNI
VTFCCKCDSTLRLCVQSTHVDIRTLEDLLMGTLGIVCPICSQKP
HPV16 E7GGG (SEQ ID NO.: 3): 
ATGCATGGAGATACACCTACATTGCATGAATATATGTTAGATTTGCAACCAGA
GACAACTGGTCTCTACGGTTATGGGCAATTAAATGACAGCTCAGAGGAGGAG
GATGAAATAGATGGTCCAGCTGGACAAGCAGAACCGGACAGAGCCCATTACA
ATATTGTAACCTTTTGTTGCAAGTGTGACTCTACGCTTCGGTTGTGCGTACAA
AGCACACACGTAGACATTCGTACTTTGGAAGACCTGTTAATGGGCACACTAG
GAATTGTGTGCCCCATCTGTTCTCAGAAACCATAA
E7GG (SEQ ID NO.: 4):

MHGDTPTLHEYMLDLQPETTGLYGYGQLNDSSEEEDEIDGPAGQAEPDRAHYNI
VTFCCKCDSTLRLCVQSTHVDIRTLEDLLMGTLGIVCPICSQKP

### Generation of thermostable carrier construct

Full length native *C*. *thermocellum* lichenase, LicB, consists sequentially of a leader peptide (Lp), an N-terminal portion (A), a surface loop (1), a C-terminal portion (C), a Pro-Thr box, and a cellulosome-binding domain (C-BD). We removed the Lp, Pro-Thr box and C-BD encoding sequences from the LicB encoding gene, circularly permutated the molecule to invert the N- and C-termini (Musiychuk et al., 2007, Influenza and Other Respiratory Viruses, 1:1), and incorporated unique restriction endonuclease sites for cloning target sequences at the new N- and C-termini as well as into the surface loop (1). The resulting engineered carrier molecule sequence was verified, and is designated LicKM.
SEQ ID NO.: 5: 
GGATCCTTAATTAAAATGGGAGGTTCTTATCCATATAAGTCTGGTGAGTATAG
AACTAAGTCTTTCTTTGGATATGGTTATTATGAAGTTAGGATGAAGGCTGCAA
AGAACGTTGGAATTGTTTCTTCTTTCTTTACTTATACTGGACCATCTGATAACA
ACCCATGGGATGAGATTGATATTGAGTTTCTTGGAAAGGATACTACTAAGGTT
CAATTCAACTGGTATAAGAATGGTGTTGGTGGAAACGAGTATCTTCATAACCT
TGGATTTGATGCTTCTCAAGATTTTCATACTTATGGTTTTGAGTGGAGACCAG
ATTATATTGATTTTTATGTTGATGGAAAGAAGGTTTATAGAGGTACTAGAAAC
ATTCCAGTTACTCCTGGAAAGATTATGATGAATCTTTGGCCAGGAATTGGTGT
TGATGAATGGCTTGGTAGATATGATGGAAGAACTCCACTTCAAGCTGAGTAT
GAGTATGTTAAGTATTATCCAAACGGTAGATCTGAATTCAAGCTTGTTGTTAA
TACTCCATTTGTTGCTGTTTTCTCTAACTTTGATTCTTCTCAATGGGAAAAGGC
TGATTGGGCTAACGGTTCTGTTTTTAACTGTGTTTGGAAGCCATCTCAAGTTA
CTTTTTCTAACGGAAAGATGATTCTTACTTTGGATAGAGAGTATGTCGACCAT
CATCATCATCATCATTGACTCGAGCTC
SEQ ID NO.: 6:

MGGSYPYKSGEYRTKSFFGYGYYEVRMKAAKNVGIVSSFFTYTGPSDNNPWDEI
DIEFLGKDTTKVQFNWYKNGVGGNEYLHNLGFDASQDFHTYGFEWRPDYIDFY
VDGKKVYRGTRNIPVTPGKIMMNLWPGIGVDEWLGRYDGRTPLQAEYEYVKYY
PNGRSEFKLVVNTPFVAVFSNFDSSQWEKADWANGSVFNCVWKPSQVTFSNGK
MILTLDREYVDHHHHHH

For certain constructs, we engineered a PRla signal peptide and KDEL sequence at the N- and C-termini of LicKM. The nucleic acid and amino acid sequences of these constructs are shown in SEQ ID NO.: 7 and SEQ ID NO.: 8.
SEQ ID NO.: 7: 
GGATCCTTAATTAAAATGGGATTTGTTCTCTTTTCACAATTGCCTTCATTTCTT
CTTGTCTCTACACTTCTCTTATTCCTAGTAATATCCCACTCTTGCCGTGCCCAA
AATGGAGGTTCTTATCCATATAAGTCTGGTGAGTATAGAACTAAGTCTTTCTT
TGGATATGGTTATTATGAAGTTAGGATGAAGGCTGCAAAGAACGTTGGAATT
GTTTCTTCTTTCTTTACTTATACTGGACCATCTGATAACAACCCATGGGATGAG
ATTGATATTGAGTTTCTTGGAAAGGATACTACTAAGGTTCAATTCAACTGGTA
 
TAAGAATGGTGTTGGTGGAAACGAGTATCTTCATAACCTTGGATTTGATGCTT
CTCAAGATTTTCATACTTATGGTTTTGAGTGGAGACCAGATTATATTGATTTTT
ATGTTGATGGAAAGAAGGTTTATAGAGGTACTAGAAACATTCCAGTTACTCCT
GGAAAGATTATGATGAATCTTTGGCCAGGAATTGGTGTTGATGAATGGCTTG
GTAGATATGATGGAAGAACTCCACTTCAAGCTGAGTATGAGTATGTTAAGTA
TTATCCAAACGGTAGATCTGAATTCAAGCTTGTTGTTAATACTCCATTTGTTGC
TGTTTTCTCTAACTTTGATTCTTCTCAATGGGAAAAGGCTGATTGGGCTAACG
GTTCTGTTTTTAACTGTGTTTGGAAGCCATCTCAAGTTACTTTTTCTAACGGAA
AGATGATTCTTACTTTGGATAGAGAGTATGTCGACCATCATCATCATCATCAT
AAGGATGAACTTTGACTCGAGCTC
SEQ ID NO.: 8:

MGFVLFSQLPSFLLVSTLLLFLVISHSCRAQNGGSYPYKSGEYRTKSFFGYGYYEV
RMKAAKNVGIVSSFFTYTGPSDNNPWDEIDIEFLGKDTTKVQFNWYKNGVGGNE
YLHNLGFDASQDFHTYGFEWRPDYIDFYVDGKKVYRGTRNIPVTPGKIMMNLW
PGIGVDEWLGRYDGRTPLQAEYEYVKYYPNGRSEFKLVVNTPFVAVFSNFDSSQ
WEKADWANGSVFNCVWKPSQVTFSNGKMILTLDREYVDHHHHHHKDEL

### Generation of recombinant antigen constructs

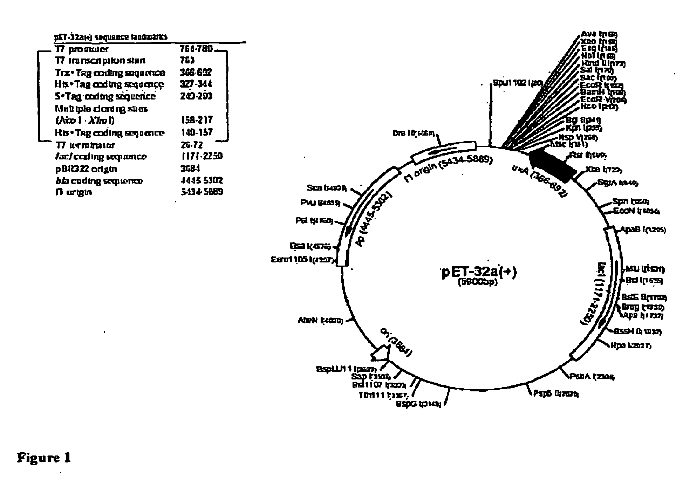

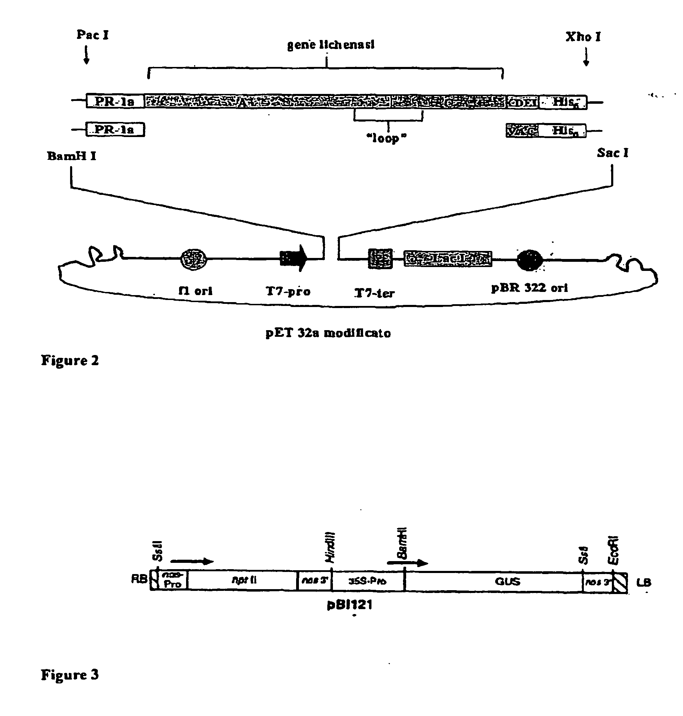

We used pET expression vectors, derived from pBR322 plasmid, engineered to take advantage of the features of the T7 bacteriophage gene 10 that promote high-level transcription and translation. The bacteriophage encoded RNA polymerase is highly specific for the T7 promoter sequences, which are rarely encountered in genomes other than T7 phage genome (Figure 1). pET-32 has been used for fusing the E7 and E7GGG constructs into the loop region of a modified lichenase sequence that had been cloned in this vector. The catalytic domain of the lichenase gene with the upstream sequence PR-1A (Pathogen-Related protein 1 A), with an endoplasmic reticulum (KDEL) or a vacuolar retaining sequence (VAC) and a downstream His6 tag were cloned between the PacI and XhoI sites in a modified pET-32 vector (in which the region between the T7 promoter and the T7 terminator had been excised). In this way the pET-PRACS-LicKM-KDEL and pET-PRACS-LicKM-VAC were obtained (Figure 2). The DNA fragment E7 or E7GGG was subcloned into the loop (1) portion of LicKM to give a fusion in the correct reading frame for translation.

### Example 2. Generation of Vaccine Candidate Antigen Vectors

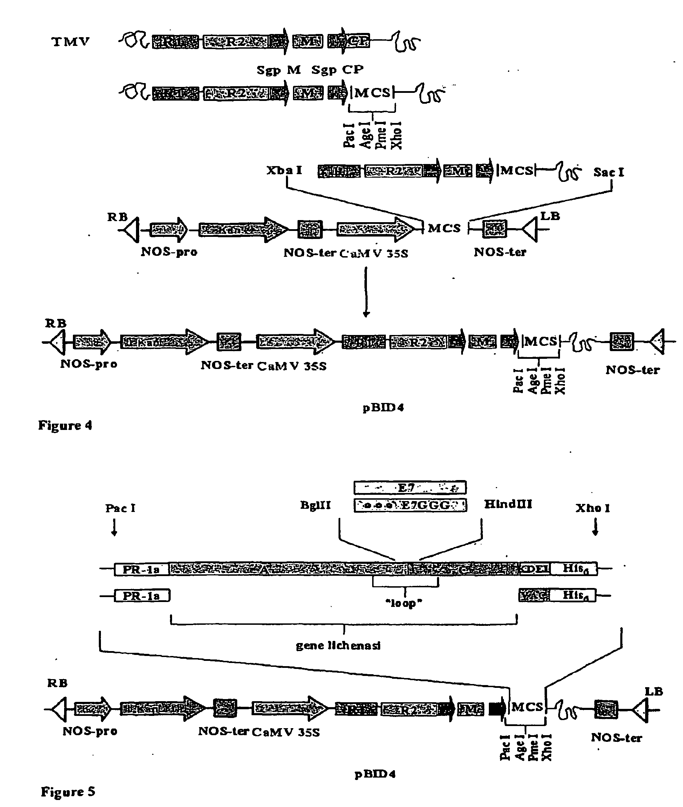

Target antigen constructs LicKM-E7 or LicKM-E7GGG were individually subcloned into the chosen viral vector (pBI-D4). pBI-D4 is a pBI121 derived binary vector in which the reporter gene coding for the *E. coli* β**-**D-glucuronidase (GUS) has been replaced by a "polylinker" where, between the XbaI and SacI sites, a TMV-derived vector has been cloned (Figure 3). pBI-D4 is a TMV-based construct in which a foreign gene to be expressed (*e.g*., target antigen (*e.g.,* LicKM-E7, LicKM-E7GG) replaces the coat protein (CP) gene of TMV. The virus retains the TMV 126/183kDa gene, the movement protein (MP) gene, and the CP subgenomic mRNA promoter (sgp), which extends into the CP open reading frame (ORF). The start codon for CP has been mutated. The virus lacks CP and therefore cannot move throughout the host plant via phloem. However, cell-to-cell movement of viral infection remains functional, and the virus can move slowly to the upper leaves in this manner. A multiple cloning site (PacI-PmeI-AgeI-XMoI) has been engineered at the end of sgp for expression of foreign genes, and is followed by the TMV 3' non-translated region (NTR). The 35S promoter is fused at the 5' end of the viral sequence. The vector sequence is positioned between the BamHI and SacI sites of pBI121. The hammerhead ribozyme is placed 3' of the viral sequence (Chen et al., 2003, Mol. Breed, 11:287). These constructs include fusions of sequences encoding LicKM-E7 or E7GGG, to sequences encoding the signal peptide from tobacco PR-la protein, a 6x His tag and the ER-retention anchor sequence KDEL or vacuolar sorting sequence (Figure 4). For constructs that contain sequence encoding, PRACS-LicKM-E7-KDEL, PRACS-LicKM-E7VAC, PRACS-LicKM-E7GGG-KDEL and PRACS-LicKM-E7GGG-VAC the coding DNA was introduced as PacI-XhoI fragments into pBI-D4. Nucleotide sequence was subsequently verified spanning the subcloning junctions of the final expression constructs (Figure 5).

### Example 3: Generation of Plants and Antigen Production

### Agrobacterium infiltration of plants

*Agrobacterium*-mediated transient expression system achieved by *Agrobacterium* infiltration can be utilized (Turpen et al., 1993, J. Virol. Methods, 42:227). Healthy leaves of *N. benthamiana* were infiltrated with *A. rhizogenes* containing viral vectors engineered to express LicKM-E7 or LicKM-E7GGG.

The *A. rhizogenes* strain A4 (ATCC 43057) or *A. tumefaciens* (GV3103) was transformed with the constructs pBI-D4- PRACS-LicKM-E7-KDEL, pBI-D4-PRACS-LicKM-E7VAC, pBI-D4-PRACS-LicKM-E7GGG-KDEL and pBI-D4-PRACS-LicKM-E7GGG-VAC. *Agrobacterium* cultures were grown and induced as described (Kapila et al., 1997, Plant Sci., 122:101) A 2 ml starter culture (picked from a fresh colony) was grown overnight in YEB (5 g/l beef extract, 1 g/l yeast extract, 5 g/l peptone, 5 g/l sucrose, 2 mM MgSO4) with 25 µg/ml kanamycin at 28oC. The starter culture was diluted 1:500 into 500 ml of YEB with 25 µg/ml kanamycin, 10 mM 2-4(-morpholino)ethanesulfonic acid (MES) pH 5.6, 2 mM additional MgSO4 and 20 µM acetosyringone. The diluted culture was then grown overnight to an O.D.600 of ~1.7 at 28oC. The cells were centrifuged at 3,000 x g for 15 minutes and re-suspended in MMA medium (MS salts, 10 mM MES pH 5.6, 20 g/l sucrose, 200 µM acetosyringone) to an O.D.600 of 2.4, kept for 1 hour at room temperature, and used *for Agrobacterium-*infiltration. *N. benthamiana* leaves were injected with the *Agrobacterium*-suspension using a disposable syringe without a needle. Infiltrated leaves were harvested 6 days post-infiltration.

### Clonal root and clonal root line generation

*Petunia hybrida* leaf explants 1 cm x 1 cm wide were obtained from leaves after sterilization in 0.1% NH4Cl and six washing in sterile dH2O. The explants, were slightly damaged with a knife on the abacsial side and co-cultured with the *Agrobacterium rhizogenes,* strain A4, containing either the pBID4-LicKM-E7-KDEL or the pBID4-LicKM-E7GGG-KDEL. The explants were incubated for 2 minutes with an *Agrobacterium* overnight culture (O.D. 600 nm=0.8-1) centrifuged for 10' at 3000 rpm a 4°C and resuspended in MMA medium to a final O.D. 600 nm=0.5 in presence of 20mM acetosyringone. At the end of the incubation, the explant were dried on sterile paper and transferred onto 0.8% agar MS plates in presence of 1% glucose and 20mM acetosyringone. Plates were parafilmed and kept at room temperature for two days. The explants were then transferred onto MS plates in presence of 500mg/l Cefotaxim (Cif), 100mg/l Timentin (Tim) and 25mg/l kanamycin. After 5 weeks the generation of transgenic roots was obtained from *Petunia hybrida* leaf explants transformed with *Agrobacterium rhizogenes* containing the pBID4-LicKM-E7-KDEL and pBID4-LicKM-E7GGG-KDEL constructs. More roots were obtained from the transformation with pBID4-LicKM-E7-KDEL than with the pBID4-LicKM-E7GGG-KDEL construct. Zymogram analysis revealed the expression of both E7 and E7GGG chimeric proteins in the *Petunia hybrida* transgenic roots tested. The expression is associated with lichenase activity. The activity band related to the fusion proteins show a higher molecular weight than the lichenase control and the same molecular weight of the product expressed by plants after agro infection, confirming the presence of whole fusion product.

After transformation, hairy roots can be cut off and placed in a line on solid, hormone free K3 medium. Four to six days later the most actively growing roots are isolated and transferred to semi-solid (0.4% agar) K3 medium. Selected roots are cultured at 22°C in the dark and clonal lines are isolated and subcultured every six weeks. Roots and/or clonal lines can be screened for the presence of target antigen expression by assessment of lichenase activity assay and immunoblot analysis.

### Example 4: Production of Vaccine Candidate

100 mg samples of *N. benthamiana* infiltrated leaf material were harvested at 4, 5, 6 and 7 days post-infection. The fresh tissue was analyzed for protein expression right after being harvested or collected at -80°C for the preparation of subsequent crude plant extracts or for fusion protein purification.

Fresh samples were resuspended in cold PBS 1x + Protease inhibitors (Roche) in a 1/3 w/v ratio (1ml / 0.3 g of tissue) and ground with a pestel. The homogenates were boiled for 5 minutes in SDS gel loading buffer and then clarified by centrifugation for 5 minutes at 12.000 rpm at 4°C. The supernatants were transferred in a fresh tube and 20 µl, 1 µl or their dilutions were separated on a 12% SDS-PAGE and analyzed by Western blot analysis using anti- His6-E7 mouse or rabbit anti-lichenase polyclonal antibodies and/or by zymogram analysis to assess hydrolytic activity indicating functional lichenase activity. Zymography is an electrophoretic method for measuring enzyme activity. The method is based on a sodium dodecyl sulfate gel impregnated with a substrate which is degraded by the enzyme resolved during the incubation period. The staining of the gel reveals enzyme activity as white bands on a dark red background. Within a certain range the band intensity can be related linearly to the amount of enzyme loaded.

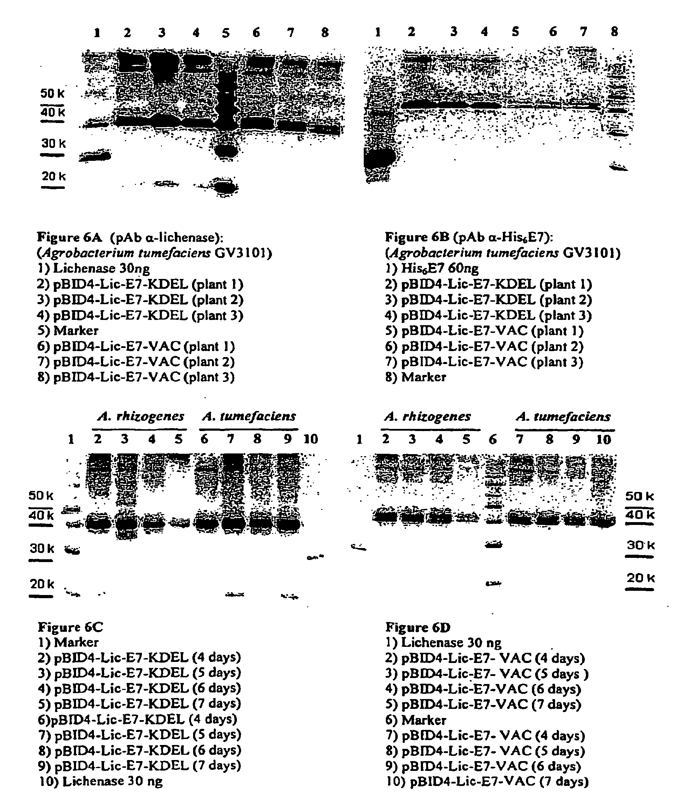

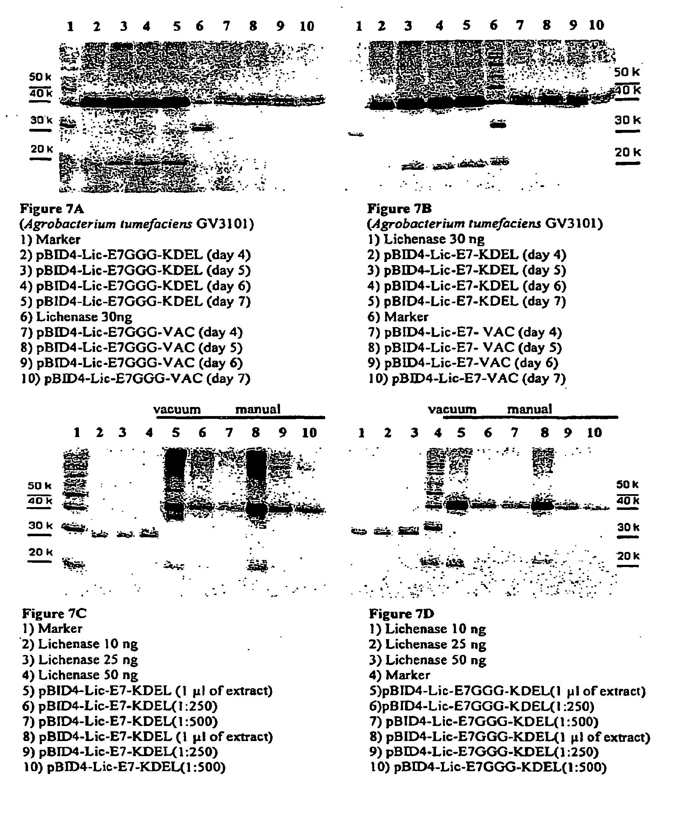

E7 expression in *Nicotiana benthamiana* plants infiltrated either with *Agrobacterium tumefaciens* or *Agrobacterium rhizogenes* containing the plasmid pBID4-LicKM-E7-KDEL leads to a specific band corresponding to the molecular weight of the chimeric protein LicKM-E7-KDEL (about 39 kD) if the E7 protein electrophoretic mobility in the fusion protein corresponds to the theoretic MW of 11 kD (the lichenase enzyme MW is about 28 kD) (Figures 6A-D). *Nicotiana benthamiana* plants infiltrated with either *Agrobacterium tumefaciens* or *Agrobacterium rhizogenes* containing the pBID4-LicKM-E7-VAC plasmids express the chimeric protein LicKM-E7-VAC. Nevertheless, the revealed band is a doublet. The double band is probably representative of the presence of both properly processed and unprocessed vacuolar signal sequence within chimeric proteins (Figures 6A-D). In both *Agrobacterium* strains the best expressing construct turned out to be the one provided with the KDEL sequence, that has been chosen for the large production of plant tissue expressing the E7 (or the E7GGG) fusion with the lichenase enzyme. The same expression results were obtained for the E7GGG chimeric proteins (Figure 7A).

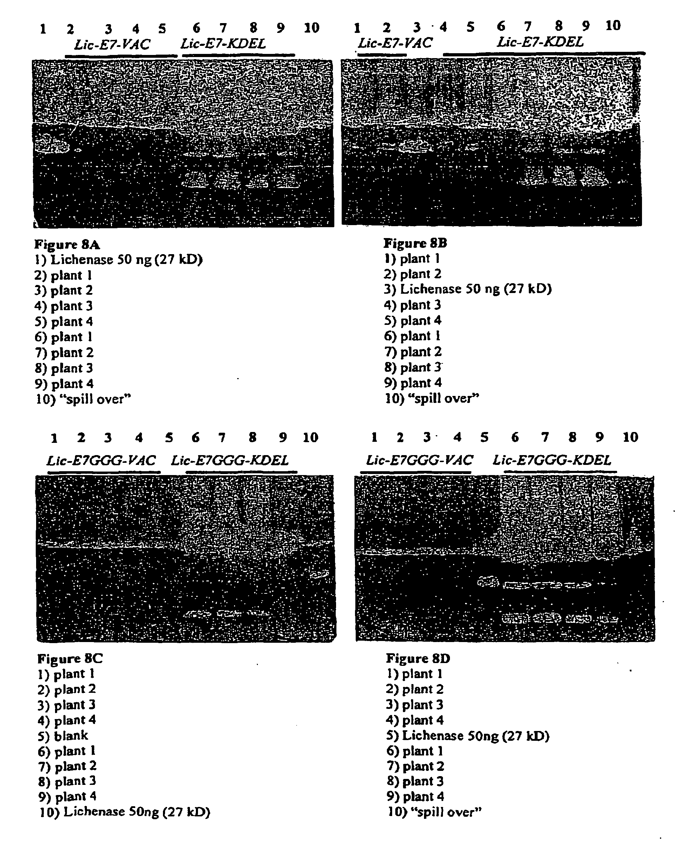

Zymogram analysis revealed that the expression of both E7 and E7GGG chimeric proteins is associated with lichenase activity, indicating the fusion of the above mentioned proteins in the loop of the catalytic domain of the lichenase enzyme doesn't impair the enzyme activity. The activity band related to the fusion proteins show a higher molecular weight than the lichenase control, confirming the presence of the whole fusion product. Extract obtained from *Nicotiana benthamiana* plants infiltrated with *Agrobocterium tumefaciens* or *Agrobacterium rhizogenes* containing pBID4-LicKM-E7-KDEL, pBID4-LicKM-E7GGG-KDEL, show an activity band, while the -VAC constructs resulted in a lesser extent of activity (Figures 8A-D).

Both the expression of the E7-LicKM-KDEL and the E7-LicKM-VAC fusions are constantly high and stable from the 4th to the 7th day post-infection (though it seems to be slightly higher at the 5th day), with the exception of the *Agrobacterium rhizogenes-*mediated expression that drops dramatically from the 6th to the 7th day. We decided to harvest at the 5th post-infection in order to avoid protein degradation. Quantification of the chimeric proteins LicKM-E7-KDEL and LicKM-E7GGG-KDEL expressed in the crude extract was made by immunoblotting both on the manually infiltrated tissues and on the vacuum-infiltrated tissues (Figures 7B,C). The estimated yield of expression in crude extract is at least 400µg of chimeric protein LicKM-E7-KDEL and LicKM-E7GGG-KDEL /g of tissue (corresponding to 100µg of E7 or E7GGG proteins / g of tissue).

### Purification of antigens

Leaves from plants infiltrated with recombinant *Agrobacterium tumefaciens* containing the pBlD4-LicKM-E7-KDEL and pBID4-LicKM-E7GGG-KDEL constructs were homogenized. Extraction buffer with "EDTA-free" protease inhibitors (Roche) and Triton X-100 1% was used at a ratio of 3 volumes w/v and rocked for 30 minutes at 4°C. Extracts were clarified by centrifugation at 9000 x g per 10 minutes at 4°C. The supernatant was sequentially filtered through Mira cloth, centrifuged at 20.000 x g for 30' at 4°C and filtered through 0.45-µm filter, before chromatographic purification.

His-tagged LicKM-E7-KDEL and LicKM-E7GGG-KDEL chimeric proteins were purified by using IMAC ("Immobilized Metal Affinity Chromatgraphy," GE Health) at room temperature under gravity. The purification was performed under non-denaturing conditions. Proteins were collected as 0.5 ml fractions, which were unified, added with 20mM EDTA, dialyzed against PBS 1X overnight at 4°C and analyzed by SDS-PAGE.

Alternatively, fractions were then collected together added with 20mM EDTA, dialyzed against NaH2PO4 10 mM, overnight, at 4°C and purified by Anion Exchange Chromatography. For LICKM-E7-KDEL E LICKM-E7GGG-KDEL purification, anion exchange column Q Sepharose Fast Flow (Amersham Pharmacia Biosciences) was used. Samples of the LICKM-E7-KDEL E LICKM-E7GGG-KDEL affinity or ion-exchange purified chimeric proteins were separated on 12% polyacrylamide gels followed by Coomassie staining. Separated proteins were also electrophoretically transferred onto PVDF membranes, and analyzed by Western blot analysis using polyclonal anti-lichenase antibody and successively with anti-rabbit IgG horseradish peroxidase-conjugated antibody.

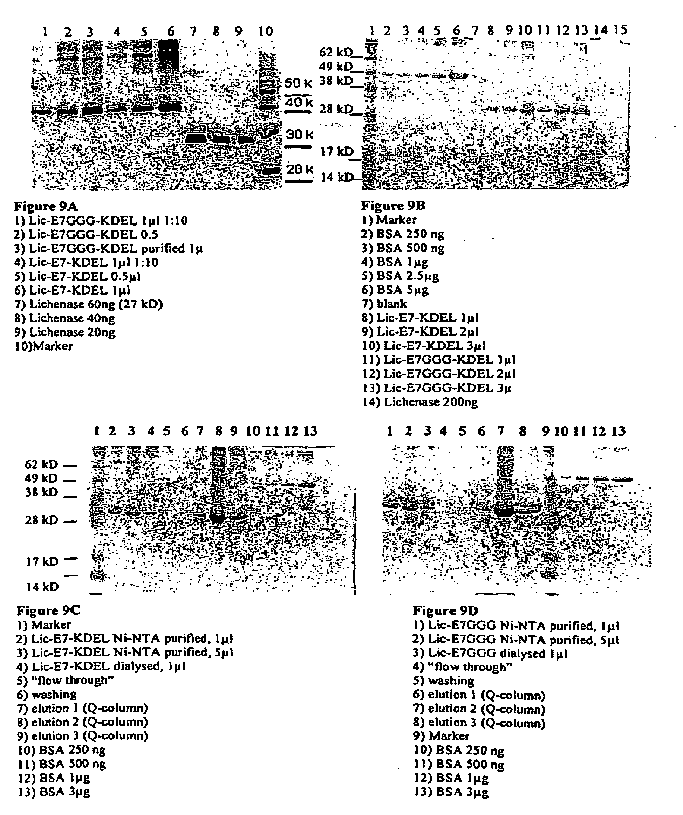

Collected fractions after dialysis were analysed by immunoblotting using both the pAb α-lichenase (Figure 9A) and the pAb α-anti-His6 (data not shown). The His-tag was maintained by the expressed chimeric proteins and the final concentration of the purified protein has been evaluated by software (Figures 9A,B). From 40 g of infiltrated tissue, 6.5 mg of each LicKM-E7-KDEL and LicKM-E7GGG-KDEL chimeric protein have been purified with a final yield of 163 µg of chimeric proteins / g of tissue (about 50 µg of E7 or E7GGG protein / g of tissue, wherein molecular weight of the E7 and of the E7GGG protein is about 1/4 of the whole fusion). Results from purification protocol are depicted in Figures 9C,D.

### Example 5: Immunogenicity Studies

### Production and characterization of target antigens

An initial immunogenicity study was conducted to determine whether plant-produced LicKM-E7-KDEL and LicKM-E7GGG-KDEL could induce specific serum IgG in mice immunized intraperitoneally, and whether the induced antibodies could bind E7-expressing cells. The study used LicKM-E7-KDEL and LicKM-E7GGG-KDEL enriched from *Agrobacterium* infiltrated leaves of *N benthamiana* to purity, as described above.

Briefly, The HPV16 E7 oncogene (GenBank accession number KO2718) was mutated using the Quikchange Site-Directed Mutagenesis Kit (Stratagene; La Jolla, CA) to give E7GGG with the following amino acid substitutions in the pRB-binding site of E7: D21G, C24G and E26G (Smahel et al., 2001, Virology, 281:231). Sequences encoding HPV 16 E7 and E7GGG were cloned as in-frame internal fusions of LicKM to obtain LicKM-E7 and LicKM-E7GGG. These fusions included the signal sequence of *Nicotiana tabacum* PR1a protein at their N-terminus, and the 6xHis tag followed by the endoplasmic reticulum retention signal KDEL at their C-terminus. The fusions were cloned in the plant expression vector pBID4 (Musiychuk et al., 2007, Influenza and Other Respiratory Viruses, 1:1, in press) to give pBID4-LicKM-E7 and pBID4-LicKM-E7GGG. Each construct was introduced into *Agrobacterium rhizogenes* strain A4 and the resulting bacteria were inoculated into *Nicotiana benthamiana.* Five to seven days post infiltration, target antigens were purified by affinity chromatography.

The estimated yields for LicKM-E7 and LicKM-E7GGG were approximately 400 mg per kg of fresh leaf tissue and the estimated yield for LicKM was approximately 1g per kg. Purified target antigens were characterized by SDS-PAGE analysis to reveal the predicted sized proteins of 28 kD (LicKM), 39 kD (LicKM-E7), and 39 kD (LicKM-E7GGG) (Fig. 9B). To provide control material, LicKM was similarly produced in *N. benthamiana.* Antigenicity of the plant-expressed proteins was verified by immunobloting using antibodies specific to LicKM and E7. The samples were quantified by densitometry using GeneTools software (Syngene; Cambridge, UK).

### Immunogenicity studies

To evaluate the efficacy of plant-produced target antigens, four to eight week old female C57BL/6 mice (Charles River; Como, Italy) were immunized with target antigens.

For prophylactic studies, 10 mice per group received 40 µg of LicKM-E7 or LicKM-E7GGG (equivalent to approximately 10 µg of E7 or E7GGG, respectively) subcutaneously, with or without Quil A adjuvant (10 µg/mouse), on days 0, 14, 28, 42 and 76. Samples of sera from animals were collected on the day of each administration and assessed for the presence of E7-specific antibodies by ELISA. On day 49, two animals in each group were sacrificed to evaluate cell mediated immune responses. All remaining animals were then challenged by subcutaneous injection with 5x10⁴ E7. expressing TC-1 tumor cells *(*Lin et al., 1996, Cancer Research, 56:21). For characterization of immune responses, ELISA, ELISPOT, and spontaneous delayed-type hypersensitivity (DTH) assays were performed as previously described (Franconi et al., 2002, Cancer Research, 62:3654).

For therapeutic studies, 8 to 10 mice per group were inoculated subcutaneously with 5x10⁴ E7-expressing TC-1 tumor cells 3 days prior to subcutaneous immunization with 40 µg LicKM-E7 or LicKM-E7GGG, with or without Quil A (10µg/mouse), on days 0, 15, 30, 45 and 60.

For both prophylactic and therapeutic studies, control animals were administered 10 µg of E. *coli*-produced E7 or E7GGG or plant-produced LicKM, with or without Quil A. Tumor growth was monitored by palpation twice a week. Throughout these studies, animals were observed for potential signs of distress, diarrhea, death or other clinical signs that may result from administration of the target antigen.

To test the immunogenicity and prophylactic potential of plant-produced LicKM-E7 and LicKM-E7GGG, animals were immunized with target antigens. In the presence of adjuvant, all animals immunized with LicKM-E7 or LicKM-E7GGG or with *E*. *coli*-produced E7 or E7GGG mounted a specific IgG response (Fig. 11A), although in the absence of adjuvant, LicKM fusions did not induce significant humoral responses (Fig. 11A). Since CD8⁺ cytotoxic T cells have a recognized role as effectors in anticancer responses, the induction of E7-specific CD8⁺ T cells was investigated by ELISPOT. In the presence of adjuvant, high numbers of IFNγ secreting cells were detected in mice vaccinated with LicKM-E7 or LicKM-E7GGG, whereas lower numbers of E7-specific CD8⁺ cells were found in mice vaccinated with E. *coli*-produced E7 or E7GGG and no IFNγ secreting cells were detected in LicKM vaccinated mice (Fig. 11B). Both LicKM fusions and E. *coli*-produced antigens induced low, but significant, numbers of spots in the absence of adjuvant, with the LicKM fusions giving the higher number of ELISPOT counts (Fig. 11B).

It has been established that HPV-specific CD8⁺ T cells are protective against challenge with an E7-expressing tumor (Frazer, 2004, Nature Reviews, 4:46). Therefore, the anti-tumor activity of the immune responses induced by plant-produced E7 candidate vaccines was evaluated by challenging vaccinated mice with TC-1 cells. In the presence of adjuvant, LicKM-E7 and LicKM-E7GGG each elicited tumor protection in 100% of animals, while *E*. *coli*-produced E7 or E7GGG induced protection in 80% and 60% of mice, respectively (Fig. 11 C). 80% of animals immunized with LicKM-E7 in the absence of adjuvant were protected (Fig. 11C). 20% of animals immunized with LicKM-E7GGG or either E. coli-produced antigen in the absence of adjuvant were protected (Fig. 11C). No protection was observed in animals immunized with LicKM (Fig. 11C).

To test for therapeutic activity of LicKM-E7 and LicKM-E7GGG against HPV16 E7-expressing tumors, animals that had been inoculated with E7-expressing TC-1 cells were subsequently immunized with LicKM, LicKM-E7, or LicKM-E7GGG, or with E. *coli*-produced E7 or E7GGG. All animals immunized with LicKM developed tumors within 4 weeks, while those treated with LicKM-E7 or LicKM-E7GGG plus adjuvant remained tumor-free for the duration of the study (10 weeks). Immunization with *E. coli-*produced E7 or E7GGG plus adjuvant inhibited tumor growth in 40% and 60% of animals, respectively (Fig. 11D). In the absence of the adjuvant, LicKM fusions inhibited tumor growth, with greater protection observed in animals that received LicKM-E7 (80%) versus LicKM-E7GGG (60%), and 20% of animals treated with E. *coli*-produced E7 or E7GGG remained tumor-free (Fig. 11D).

Protection against the development of HPV-associated disease is in large attributed to the cell-mediated immune responses. The DTH response is thought to represent antigen-specific cytokine mediated inflammation, particularly involving Th1 type cytokines. Since LicKM-E7 showed greater therapeutic activity than LicKM-E7GGG against E7-expressing tumors, we evaluated the DTH response to E7 in mice vaccinated with LicKM-E7 or *E. coli*-produced E7. An antigen-specific DTH response was observed in mice vaccinated with the LicKM-E7 protein, even in the absence of adjuvant (Table 1). This response exceeded that observed in mice vaccinated with E. *coli*-produced E7. Mice immunized with LicKM showed no significant ear swelling, demonstrating that the LicKM carrier molecule does not induce an inflammatory response.

**Table 1. Delayed Type Hypersensitivity**

| | **Δ ear thickness* ± standard deviation** | |
|---|---|---|
| | **48 hours** | **72 hours** |
| LicKM | 2.5 ± 0.7 | 1.0 ± 0.7 |
| *E. coli* E7 | 2.3 + 1.5 | 4.6 ± 2.5 |
| LicKM-E7 | 5.0 ± 2.7 | 10 + 2.3 |
| LicKM-E7 + Quil A | 6.5 ± 0.7 | 7.0 ± 0.1 |

| | | |
|---|---|---|
| * ear swelling was reported as the mean of the differences (Δ) in thickness between challenged and unchallenged control ears from 5 mice per group (mm ear thickening x 10⁻²). | | |

Prior art document Smahel M. et al., The Journal of Gene Medicine, vol. 6, no. 10, 2004-10-01, pages 1092-1101, describes enhancement of immunogenicity of HPV16 E7 oncogene by fusion with E. coli beta-glucuronidase.
Document WO 2005/026375 discloses carrier molecules, in particular a modified beta 1,3-1,4-glucanase from Chlostridium thermocellum, LicKM, for expression of a target polypeptide fused to LicKM for stimulating an immune response.

## Claims

1. An isolated antigen comprising a component of human papilloma virus (HPV) fused to a LicKM protein;
wherein the HPV component comprises at least one domain selected from the group consisting of E7 from HPV16, E7 from HPV 18, E6 from HPV 16, and E6 from HPV 18, a fragment of E7 from HPV 16, a fragment of E7 from HPV 18, a fragment of E6 from HPV16, and a fragment of E6 from HPV 18, and
wherein the LicKM protein comprises the amino acid sequence of SEQ ID NO:6 or SEQ ID NO:8.

2. The isolated antigen of claim 1, wherein the HPV component consists of E7 from HPV 16 or HPV18.

3. The isolated antigen of claim 1, wherein the HPV component consists of SEQ ID NO.: 2 or SEQ ID NO.: 4.

4. The isolated antigen of claim 1, wherein the HPV component is fused to the LicKM protein as an N-terminal fusion, a C-terminal fusion, or a surface loop insertion fusion.

5. The isolated antigen of claim 1, wherein the HPV component comprises at least two domains independently selected from the group consisting of E7 from HPV16, E7 from HPV18, E6 from HPV16, and E6 from HPV18, a fragment of E7 from HPV16, a fragment of E7 from HPV 18, a fragment of E6 from HPV16, and a fragment of E6 from HPV18.

6. A vaccine composition comprising the antigen of any one of claims 1 to 5 and a pharmaceutically acceptable carrier and wherein the composition is capable of eliciting an immune response against HPV infection upon administration to a subject. infection

7. The vaccine composition of claim 6, further comprising a second antigen, wherein the second antigen comprises a component of human papilloma virus (HPV) fused to a LicKM protein and a pharmaceutically acceptable carrier; wherein the HPV component of the first antigen comprises at least one domain selected from the group consisting of E7 from HPV16, E7 from HPV 18, E6 from HPV16, and E6 from HPV 18, a fragment of E7 from HPV16, a fragment of E7 from HPVIS, a fragment of E6 from HPV 16, and a fragment of E6 from HPV18, and the HPV component of the second antigen comprises at least one domain distinct from the first antigen selected from the group consisting of E7 from HPV 16, E7 from HPV 18, E6 from HPV 16, and E6 from HPV 18, a fragment of E7 from HPV 16, a fragment of E7 from HPVIS, a fragment of E6 from HPV 16, and a fragment of E6 from HPV18.

8. The vaccine composition of claim 6, wherein the antigen is produced in a plant selected from a transgenic plant and a plant transiently expressing the antigen.

9. The vaccine composition of claim 6, wherein the composition comprises antigen which is purified, partially purified, or unpurified from plant cells, a plant, seeds, fruit, or an extract thereof.

10. The vaccine composition of claim 6, further comprising a vaccine adjuvant, for example alum, MF59, MALP2, or saponin.

11. A vaccine composition comprising at least two antigens, each of which comprises a component of human papilloma virus (HPV), wherein at least one of the antigens further comprises a LicKM protein fused to the HPV component, wherein the composition is capable of eliciting an immune response against HPV infection upon administration to a subject, and wherein the LicKM protein comprises the amino acid sequence of SEQ ID NO:6 or SEQ ID NO:8.

12. The vaccine composition of claim 11, wherein at least two antigens comprises a component of HPV, each of which independently comprises at least one domain selected from the group consisting of E7 from HPV16, E7 from HPV 18, E6 from HPV16, and E6 from HPV 18, a fragment of E7 from HPV16, a fragment of E7 from HPV18, a fragment of E6 from HPV16, and a fragment of E6 from HPV 18 and combinations thereof.

13. An anti-HPV vaccine composition for use in inducing a cytotoxic T cell response against human papilloma virus (HPV) infection in a subject;
a. wherein the vaccine composition comprises antigen comprising a component of human papilloma virus (HPV) fused to a LicKM protein, wherein the LicKM protein comprises the amino acid sequence of SEQ ID NO:6 or SEQ ID NO:8; and
b. wherein the HPV component comprises at least one domain at least one domain selected from the group consisting of E7 from HPV16, E7 from HPV18, E6 from HPV16, and E6 from HPV18, a fragment of E7 from HPV16, a fragment of E7 from HPV18, a fragment of E6 from HPV16, and a fragment of E6 from HPV 18.

14. The composition of claim 13, wherein the composition is for administration orally, for example via feeding plant cells to the subject, intranasally, subcutaneously, intravenously, intraperitoneally, or intramuscularly.

15. The composition of claim 13, wherein the subject is human.

16. A method for producing an antigen protein comprising a component of human papilloma virus (HPV) fused to a LicKM protein, comprising:
a. preparing a nucleic acid construct encoding an antigen component of human papilloma virus (HPV) fused to a LicKM protein, wherein the LicKM protein comprises the amino acid sequence of SEQ ID NO:6 or SEQ ID NO:8;
b. transforming a plant cell with the nucleic acid construct of step a; and
c. incubating the plant cell under conditions favorable for expression of the antigen protein;
d. thereby producing the antigen protein;
wherein the HPV component of the antigen comprises at least one domain selected from the group consisting of selected from the group consisting of E7 from HPV 16, E7 from HPV18, E6 from HPV 16, and E6 from HPV 18, a fragment of E7 from HPV 16, a fragment of E7 from HPV18, a fragment of E6 from HPV16, and a fragment of E6 from HPV18.

17. The method of claim 16, wherein the HPV component consists of E7 from HPV16 or HPV 18.

18. The method of claim 16, wherein the HPV component is encoded by a nucleotide sequence selected from the group consisting of SEQ ID NO.: 1 and SEQ NO.: 3.

19. The method of claim 16, wherein the HPV component consists of SEQ ID NO.: 2 or SEQ ID NO.: 4.

20. The method of claim 16, wherein the HPV component is fused to the LicKM protein as an N-terminal fusion, a C-terminal fusion, or a surface loop insertion fusion.

21. The method of claim 16, wherein the HPV component comprises at least two domains each of which independently comprises at least one domain selected from the group consisting of E7 from HPV16, E7 from HPV 18, E6 from HPV16, and E6 from HPV 18, a fragment of E7 from HPV16, a fragment of E7 from HPV 18, a fragment of E6 from HPV16, and a fragment of E6 from HPV18 and combinations thereof.

22. The method of claim 16, wherein expression of the antigen protein is under control of a viral promoter.

23. The method of claim 16, wherein the nucleic acid construct further comprises vector nucleic acid sequence and/or the nucleic acid construct further comprises sequences encoding viral proteins.

24. The method of claim 23, wherein the vector is a binary vector.

25. The method of claim 16, wherein the plant cell is selected from the group consisting of alfalfa, radish, mustard, mung bean, broccoli, watercress, soybean, wheat sunflower, cabbage, clover, petunia, tomato, potato, nicotine, spinach, and lentil cell.

26. The method of claim 25, wherein the antigen protein is produced in a clonal root cell and/or in sprouted seedlings.

27. The method of claim 16, further comprising recovering partially purified or purified antigen protein which is produced.

28. A plant of a genus selected from the *Brassica* genus, the *Nicotiana* genus, and the *Petunia* genus comprising a nucleic acid sequence encoding a component of human papilloma virus (HPV) fused to a LicKM protein;
wherein the HPV component comprises at least one domain selected from the group consisting of E7 from HPV 16, E7 from HPV 18, E6 from HPV 16, and E6 from HPV18, a fragment of E7 from HPV 16, a fragment of E7 from HPV18, a fragment of E6 from HPV 16, and a fragment of E6 from HPV 18 and combinations thereof,
wherein the LicKM protein comprises the amino acid sequence of SEQ ID NO:6 or SEQ ID NO:8; and
wherein the plant is capable of producing the fusion protein.

## Patentansprüche

1. Isoliertes Antigen umfassend eine an ein LicKM-Protein fusionierte Komponente des humanen Papillomavirus (HPV),
wobei die HPV-Komponente mindestens eine Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18, und
wobei das LicKM-Protein die Aminosäuresequenz gemäß SEQ ID NR: 6 oder SEQ ID NR: 8 umfasst.

2. Isoliertes Antigen nach Anspruch 1, wobei die HPV-Komponente aus E7 aus HPV16 oder HPV18 besteht.

3. Isoliertes Antigen nach Anspruch 1, wobei die HPV-Komponente aus SEQ ID NR: 2 oder SEQ ID NR: 4 besteht.

4. Isoliertes Antigen nach Anspruch 1, wobei die HPV-Komponente mittels einer N-terminalen Fusion, einer C-terminalen Fusion oder einer Surface-Loop-Insertionsfusion an das LicKM-Protein fusioniert ist.

5. Isoliertes Antigen nach Anspruch 1, wobei die HPV-Komponente mindestens zwei Domänen umfasst, die jeweils unabhängig voneinander ausgewählt sind aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18.

6. Vakzinenzusammensetzung, die das Antigen nach einem der Ansprüche 1 bis 5 sowie einen pharmazeutisch unbedenklichen Träger umfasst, wobei die Zusammensetzung bei der Verabreichung an ein Subjekt zur Induktion einer Immunantwort auf eine HPV-Infektion in der Lage ist.

7. Vakzinenzusammensetzung nach Anspruch 6, die des Weiteren ein zweites Antigen umfasst, wobei das zweite Antigen eine an ein LicKM-Protein fusionierte Komponente des humanen Papillomavirus (HPV) sowie einen pharmazeutisch unbedenklichen Träger umfasst; wobei die HPV-Komponente des ersten Antigens mindestens eine Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18, und die HPV-Komponente des zweiten Antigens mindestens eine von dem ersten Antigen verschiedene Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18.

8. Vakzinenzusammensetzung nach Anspruch 6, wobei das Antigen in einer Pflanze produziert wird, die ausgewählt ist aus einer transgenen Pflanze und einer Pflanze, die das Antigen transient exprimiert.

9. Vakzinenzusammensetzung nach Anspruch 6, wobei die Zusammensetzung Antigen umfasst, das aufgereinigt, partiell aufgereinigt oder nicht aufgereinigt aus Pflanzenzellen, einer Pflanze, Samen, Früchten oder Extrakten der Pflanze erhalten ist.

10. Vakzinenzusammensetzung nach Anspruch 6, des Weiteren umfassend ein Vakzinenadjuvans, wie z. B. Alaun, MF59, MALP2 oder Saponin.

11. Vakzinenzusammensetzung, die mindestens zwei Antigene umfasst, von denen ein jedes eine Komponente des humanen Papillomavirus (HPV) umfasst, wobei mindestens eines der Antigene des Weiteren ein an die HPV-Komponente fusioniertes LicKM-Protein umfasst, wobei die Zusammensetzung bei der Verabreichung an ein Subjekt zur Induktion einer Immunantwort auf eine HPV-Infektion in der Lage ist und wobei das LicKM-Protein die Aminosäuresequenz gemäß SEQ ID NR: 6 oder SEQ ID NR: 8 umfasst.

12. Vakzinenzusammensetzung nach Anspruch 11, wobei mindestens zwei Antigene eine Komponente des HPV umfassen, die jeweils unabängig mindestens eine Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18 sowie Kombinationen aus diesen.

13. Anti-HPV-Vakzinenzusammensetzung zur Verwendung bei der Induktion einer zytotoxischen T-Zellantwort gegen eine Infektion mit dem humanen Papillomavirus (HPV) in einem Subjekt;
a. wobei die Vakzinenzusammensetzung Antigen umfasst, das eine an ein LicKM-Protein fusionierte Komponente des humanen Papillomavirus (HPV) umfasst, wobei das LicKM-Protein die Aminosäuresequenz gemäß SEQ ID NR: 6 oder SEQ ID NR: 8 umfasst; und
b. wobei die HPV-Komponente mindestens eine Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18.

14. Zusammensetzung nach Anspruch 13, wobei die Zusammensetzung zur oralen, wie z. B. über die Ingestion von Pflanzenzellen durch das Subjekt, intranasalen, subkutanen, intravenösen, intraperitonealen oder intramuskulären Verabreichung vorgesehen ist.

15. Zusammensetzung nach Anspruch 13, wobei es sich bei dem Subjekt um einen Menschen handelt.

16. Verfahren zur Herstellung eines Antigenproteins, das eine an ein LicKM-Protein fusionierte Komponente des humanen Papillomavirus (HPV) umfasst, umfassend:
a. das Herstellen eines Nukleinsäurekonstrukts, das für eine an ein LicKM-Protein fusionierte Antigenkomponente des humanen Papillomavirus (HPV) kodiert, wobei das LicKM-Protein die Aminosäuresequenz gemäß SEQ ID NR: 6 oder SEQ ID NR: 8 umfasst;
b. das Transformieren einer Pflanzenzelle mit dem Nukleinsäurekonstrukt aus Schritt a;
c. das Inkubieren der Pflanzenzelle unter Bedingungen, die eine Expression des Antigenproteins begünstigen; und
d. das dadurch erfolgende Herstellen des Antigenproteins;
wobei die HPV-Komponente des Antigens mindestens eine Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18.

17. Verfahren nach Anspruch 16, wobei die HPV-Komponente aus E7 aus HPV16 oder HPV18 besteht.

18. Verfahren nach Anspruch 16, wobei die HPV-Komponente durch eine Nukleotidsequenz kodiert wird, die ausgewählt ist aus der Gruppe bestehend aus SEQ ID NR: 1 und SEQ ID NR: 3.

19. Verfahren nach Anspruch 16, wobei die HPV-Komponente aus SEQ ID NR: 2 oder SEQ ID NR: 4 besteht.

20. Verfahren nach Anspruch 16, wobei die HPV-Komponente mittels einer N-terminalen Fusion, einer C-terminalen Fusion oder einer Surface-Loop-Insertionsfusion an das LicKM-Protein fusioniert ist.

21. Verfahren nach Anspruch 16, wobei die HPV-Komponente mindestens zwei Domänen umfasst, die jeweils unabhängig voneinander mindestens eine Domäne umfassen, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18 und Kombinationen aus diesen.

22. Verfahren nach Anspruch 16, wobei die Expression des Antigenproteins unter der Kontrolle eines viralen Promotors steht.

23. Verfahren nach Anspruch 16, wobei das Nukleinsäurekonstrukt des Weiteren eine Vektornukleinsäuresequenz umfasst und/oder das Nukleinsäurekonstrukt des Weiteren für virale Proteine kodierende Sequenzen umfasst.

24. Verfahren nach Anspruch 23, wobei es sich bei dem Vektor um einen binären Vektor handelt.

25. Verfahren nach Anspruch 16, wobei die Pflanzenzelle ausgewählt ist aus der Gruppe bestehend aus Alfalfa, Rettich, Senf, Mungbohne, Brokkoli, Brunnenkresse, Sojabohne, Weizen, Sonnenblume, Kohl, Klee, Petunie, Tomate, Kartoffel, Nikotin, Spinat und Linse.

26. Verfahren nach Anspruch 25, wobei das Antigenprotein in einer klonalen Wurzelzelle und/oder in gekeimten Sämlingen produziert wird.

27. Verfahren nach Anspruch 16, des Weiteren umfassend das Gewinnen von partiell aufgereinigtem oder aufgereinigtem produziertem Antigenprotein.

28. Pflanze einer Gattung, die ausgewählt ist aus der Gattung *Brassica,* der Gattung *Nicotiana* und der Gattung *Petunia,* umfassend eine Nukleinsäuresequenz, die für eine an ein LicKM-Protein fusionierte Komponente des humanen Papillomavirus (HPV) kodiert;
wobei die HPV-Komponente mindestens eine Domäne umfasst, die ausgewählt ist aus der Gruppe bestehend aus E7 aus HPV16, E7 aus HPV18, E6 aus HPV16 und E6 aus HPV18, einem Fragment von E7 aus HPV16, einem Fragment von E7 aus HPV18, einem Fragment von E6 aus HPV16 und einem Fragment von E6 aus HPV18 und Kombinationen aus diesen,
wobei das LicKM-Protein die Aminosäuresequenz gemäß SEQ ID NR: 6 oder SEQ ID NR: 8 umfasst; und
wobei die Pflanze zur Produktion des Fusionsproteins in der Lage ist.

## Revendications

1. Un antigène isolé comprenant un composant du virus du papillome humain (HPV) fusionné à une protéine LicKM; dans lequel le composant du HPV comprend au moins un domaine choisi dans le groupe consistant en E7 provenant de HPV16, E7 provenant de HPV18, E6 provenant de HPV 16, et E6 provenant de HPV18, un fragment de E7 provenant de HPV 16, un fragment de E7 provenant de HPV18, un fragment de E6 provenant de HPV16, et un fragment de E6 provenant de HPV18, et dans lequel la protéine LicKM comprend la séquence d'acide aminé de SEQ ID NO:6 ou SEQ ID NO:8.

2. L'antigène isolé de la revendication 1, dans lequel le composant du HPV est constitué de E7 provenant de HPV1 6 ou HPV1 8.

3. L'antigène isolé de la revendication 1, dans lequel le composant du HPV consiste en SEQ ID NO:2 ou SEQ ID NO:4.

4. L'antigène isolé de la revendication 1, dans lequel le composant du HPV est fusionné à la protéine LicKM en tant qu'une fusion N-terminale, une fusion C-terminale, ou une fusion par insertion de boucle de surface.

5. L'antigène isolé de la revendication 1, dans lequel le composant du HPV comprend au moins deux domaines choisis indépendamment dans le groupe consistant en E7 provenant de HPV16, E7 provenant du HPV18, E6 provenant de HPV16, et E6 provenant de HPV18, un fragment de E7 provenant du HPV16, un fragment de E7 provenant du HPV18, un fragment de E6 provenant de HPV16, et un fragment de E6 provenant de HPV18.

6. Une composition de vaccin comprenant l'antigène de l'une quelconque des revendications 1 à 5, et un support acceptable sur le plan pharmaceutique, et dans laquelle la composition est capable de provoquer une réponse immunitaire contre une infection par le HPV lors d'une administration à un sujet.

7. La composition de vaccin de la revendication 6, comprenant en outre un second antigène, dans laquelle le second antigène comprend un composant du virus du papillome humain (HPV) fusionné à une protéine LicKM et un support acceptable sur le plan pharmaceutique; dans laquelle le composant de HPV du premier antigène comprend au moins un domaine choisi dans le groupe consistant en E7 provenant de HPV16, E7 provenant du HPV18, E6 provenant du HPV16, et E6 provenant de HPV18, un fragment de E7 provenant de HPV16, un fragment de E7 provenant de HPV18, un fragment de E6 provenant de HPV16, et un fragment de E6 provenant de HPV18, et le composant de HPV du second antigène comprend au moins un domaine distinct du premier antigène sélectionné dans le groupe consistant en E7 provenant de HPV16, E7 provenant du HPV18, E6 provenant du HPV16, et E6 provenant de HPV18, un fragment de E7 provenant de HPV16, un fragment de E7 provenant de HPV18, un fragment de E6 provenant de HPV16, et un fragment de E6 provenant du HPV18.

8. La composition du vaccin de la revendication 6, dans laquelle l'antigène est produit dans une plante sélectionnée à partir d'une plante transgénique et d'une plante exprimant transitoirement l'antigène.

9. La composition de vaccin de la revendication 6, dans laquelle la composition comprend un antigène qui est purifié, partiellement purifié ou non purifié à partir de cellules de plantes, d'une plante, de semences, de fruit, ou d'un extrait de ceux-ci.

10. La composition de vaccin de la revendication 6, comprenant en outre un adjuvant de vaccin, par exemple de l'alun, MF59, MALP2, ou de la saponine.

11. Une composition de vaccin comprenant au moins deux antigènes, dont chacun comprend un composant du virus du papillome humain (HPV), dans laquelle au moins l'un des antigènes comprend en outre une protéine LicKM fusionnée au composant HPV, dans laquelle la composition est capable de provoquer une réponse immunitaire contre une infection HPV lors de l'administration à un sujet, et dans laquelle la protéine LicKM comprend la séquence d'acide aminé de SEQ ID NO:6 ou SEQ ID NO:8.

12. La composition de vaccin de la revendication 11, dans lequel au moins deux antigènes comprennent un composant de HPV dont chacun comprend indépendamment au moins un domaine choisi dans le groupe consistant en E7 provenant de HPV16, E7 provenant du HPV18, E6 provenant du HPV16, et E6 provenant de HPV18, un fragment de E7 provenant de HPV16, un fragment de E7 provenant de HPV18, un fragment de E6 provenant de HPV16, et un fragment de E6 provenant de HPV18 et des combinaisons de ceux-ci.

13. Une composition de vaccin contre le HPV pour un usage pour induire une réponse de cellule T cytotoxique contre une infection par le virus du papillome humain (HPV) chez un sujet;
a. dans laquelle la composition de vaccin comprend un antigène comprenant un composant de virus du papillome humain (VPH) fusionné à une protéine LicKM, dans laquelle la protéine LicKM comprend la séquence d'acide aminé de SEQ ID NO:6 ou SEQ ID NO:8; et
b. dans laquelle le composant du HPV comprend au moins un domaine au moins un domaine choisi dans le groupe consistant en E7 provenant de HPV16, E7 provenant du HPV18, E6 provenant du HPV16, et E6 provenant de HPV18, un fragment de E7 provenant de HPV16, un fragment de E7 provenant de HPV18, un fragment de E6 provenant de HPV16, et un fragment de E6 provenant de HPV1 8.

14. La composition de la revendication 13, dans laquelle la composition est destinée à une administration par voie orale, par exemple en alimentant le sujet avec des cellules de plante, une administration intra-nasale, sous-cutanée, intraveineuse, intra-péritonéale ou intramusculaire.

15. La composition de la revendication 13, dans laquelle le sujet est un humain.

16. Un procédé de production d'une protéine antigénique comprenant un composant de virus du papillome humain (HPV) fusionné à une protéine LicKM, comprenant:
a. la préparation d'une construction d'acide nucléique codant pour un composant antigène du virus du papillome humain (HPV) fusionné à une protéine LicKM, dans laquelle la protéine LicKM comprend la séquence d'acide aminé de SEQ ID NO:6 ou SEQ ID NO:8;
b. la transformation d'une cellule de plante au moyen de la construction d'acide nucléique de l'étape a; et
c. l'incubation de la cellule de plante dans des conditions favorables pour l'expression de la protéine antigénique;
d. en produisant ainsi la protéine antigénique;
dans lequel le composant de HPV de l'antigène comprend au moins un domaine choisi dans le groupe constitué par choisi dans le groupe constitué par E7 provenant de HPV16, E7 provenant du HPV18, E6 provenant du HPV16, et E6 provenant de HPV18, un fragment de E7 provenant de HPV16, un fragment de E7 provenant de HPV18, un fragment de E6 provenant de HPV16, et un fragment de E6 provenant de HPV1 8.

17. Le procédé de la revendication 16, dans lequel le composant de HPV est constitué E7 provenant de HPV16 ou HPV1 8.

18. Le procédé de la revendication 16, dans lequel le composant de HPV est codé par une séquence nucléotidique choisie dans le groupe constitué de SEQ ID NO:1 et SEQ ID NO:3.

19. Le procédé de la revendication 16, dans lequel le composant de HPV consiste en SEQ ID NO:2 ou SEQ ID NO:4.

20. Le procédé de la revendication 16, dans lequel le composant de HPV est fusionné à la protéine LicKM sous forme d'une fusion N-terminale, une fusion C-terminale, ou une fusion par insertion de boucle de surface.

21. Le procédé de la revendication 16, dans lequel le composant de HPV comprend au moins deux domaines dont chacun comprend indépendamment au moins un domaine choisi dans le groupe consistant en E7 provenant de HPV16, E7 provenant du HPV18, E6 provenant du HPV16, et E6 provenant de HPV18, un fragment de E7 provenant de HPV 16, un fragment de E7 provenant de HPV18, un fragment de E6 provenant de HPV16, et un fragment de E6 provenant de HPV1 8 et des combinaisons de ceux-ci.

22. Le procédé de la revendication 16, dans lequel l'expression de la protéine antigénique est sous le contrôle d'un promoteur viral.

23. Le procédé de la revendication 16, dans lequel la construction d'acide nucléique comprend en outre une séquence de vecteur d'acide nucléique et/ou la construction d'acide nucléique comprend en outre des séquences codant pour des protéines virales.

24. Le procédé de la revendication 23, dans lequel le vecteur est un vecteur binaire.

25. Le procédé de la revendication 16, dans lequel la cellule de plante est choisie dans le groupe constitué de la cellule de luzerne, de radis, de moutarde, de haricot mungo, de brocoli, de cresson, de soja, de blé, de tournesol, de chou, de trèfle, de pétunia, de tomate, de pomme de terre, de nicotine, d'épinard, et de lentille.

26. Le procédé de la revendication 25, dans lequel la protéine antigénique est produite dans un cellule de racine clonale et/ou dans des plants germés.

27. Le procédé de la revendication 16, comprenant en outre la récupération de la protéine antigénique partiellement purifiée ou purifiée qui est produite.

28. Une plante d'un genre choisi parmi le genre *Brassica,* le genre *Nicotiana* et le genre *Petunia* comprenant une séquence d'acide nucléique codant pour un composant du virus du papillome humain (HPV) fusionné à une protéine LicKM;
dans lequel le composant du HPV comprend au moins un domaine choisi dans le groupe consistant en E7 provenant de HPV16, E7 provenant de HPV18, E6 provenant de HPV 16, et E6 provenant de HPV18, un fragment de E7 provenant de HPV 16, un fragment de E7 provenant de HPV18, un fragment de E6 provenant de HPV16, et un fragment de E6 provenant de HPV18, et des combinaisons de ceux-ci,
dans lequel la protéine LicKM comprend la séquence d'acide aminé de SEQ ID NO:6 ou SEQ ID NO:8 ; et
où la plante est capable de produire la protéine de fusion.
